Europäisches Patentamt

European Patent Office

Office européen des brevets

(11) Publication number: 0 370 711
A1

## (12) EUROPEAN PATENT APPLICATION

(21) Application number: 89311952.9

(22) Date of filing: 17.11.89

(51) Int. Cl.⁵: **C12N 9/64, C12N 9/68, C12N 15/58, A61K 37/547, C12N 15/57, C12N 15/62**

(30) Priority: 18.11.88 GB 8826975

(43) Date of publication of application:
30.05.90 Bulletin 90/22

(84) Designated Contracting States:
BE CH DE FR GB IT LI NL

(71) Applicant: BEECHAM GROUP PLC
Beecham House Great West Road
Brentford Middlesex TW8 9BD(GB)

(72) Inventor: Browne, Michael Joseph Beecham
Pharmaceuticals
Biosciences Research Centre Great Burgh
Yew Tree Bottom Rd Epsom Surrey KT18
5XQ(GB)
Inventor: Dodd, Ian Beecham
Pharmaceuticals
Biosciences Research Centre Great Burgh
Yew Tree Bottom Rd Epsom Surrey KT18
5XQ(GB)
Inventor: Robinson, Jeffery Hugh Beecham
Pharmaceuticals
Biosciences Research Centre Great Burgh
Yew Tree Bottom Rd Epsom Surrey KT18
5XQ(GB)

(74) Representative: Valentine, Jill Barbara et al
Beecham Pharmaceuticals Patents & Trade
Marks Dept. Great Burgh Yew Tree Bottom
Road
Epsom Surrey KT18 5XQ(GB)

(54) Novel compounds.

(57) Plasminogen or a plasminogen activator containing the five kringle domains or plasminogen in which a carbohydrate structure cannot be attached to amino acid 289.

## Novel Compounds

The present invention relates to plasminogen and plasminogen activators containing the five kringle domains of plasminogen, their preparation, pharmaceutical compositions containing them and their use in the treatment of thrombotic disease, in particular acute myocardial infarction.

Plasmin is a two-chain serine protease which may be obtained by the cleavage of the single chain precursor, plasminogen, at a specific internal peptide bond. The amino acid sequence of human plasminogen is known (Wiman and Walters (1975) Eur.J. Biochem. 50, 489-494 and 58, 539-547; Wiman (1977) Eur. J. Biochem. 76, 129-137; Sottrup-Jensen et al. (1978) Fibrinolysis and Thrombolysis Vol. 3, 191-209, Raven Press, New York; and Sottrup-Jensen et al. (1978) Atlas of Protein Sequence and Structure Vol. 5, Suppl. 3, p91, National Biomedical Research Foundation, Silver Spring, MD). A partial nucleotide sequence coding for amino acid residues 272-790 of human plasminogen has also been described (Malinowski, D.P. et al., 1984, Biochemistry, 23, 4243-4250). The cleavage site of human plasminogen is located between residues arg-560 and val-561 (according to the sequence numbering of Sottrup-Jensen et al. (1978) Atlas of Protein Sequence (op.cit.)). Two species of plasminogen have been identified ( F.J. Castellino, Chemical Reviews Vol. 81 p431 (1981)): $glu_1$ which has an N-terminal glutamic acid residue at position 1 and $lys_{77}$ which has an N-terminal lysine residue at position 77. Glu-plasminogen is also easily converted by limited plasmin digestion to other modified forms with N-terminal valine ($val_{78}$) or methionine ($met_{68}$) (C. Miyashita, E. Wenzel and M. Heiden, Haemostasis 18, supp.1 pp 7-13 (1988)). References to plasminogen herein are understood to include all these species.

Recently a complete nucleotide sequence has been described (Forsgren, M., et al., 1987, FEBS Letters 213, 254-260). The nucleotide sequence predicts the existence of an extra, previously unreported, isoleucine residue near the N-terminus of the A-chain. This finding has been independently confirmed (McLean, J.N., et al., 1987, Nature 330, 132-137). Accordingly all sequence numbering (amino acid and nucleotide) below follows Forsgren et al. (1987). In this numbering sequence the plasminogen cleavage site is located between residues arg-561 and val-562 and the N-terminal modified forms are termed $met_{69}$, $lys_{78}$ and $val_{79}$.

Plasminogen has five kringle structures. The region from the first to the last cysteine residue of each kringle structure, residues 84 to 162, 166 to 243, 256 to 333, 358 to 435 and 462 to 541 inclusive will be referred to herein as the $K_1^P$, $K_2^P$, $K_3^P$, $K_4^P$ and $K_5^P$ domains respectively.

Human plasminogen has two glycosylation sites: asparagine at position 289 in kringle 3 and threonine at position 346 between kringles 3 and 4 (Hayes, M.L. and Castellino, F.J., J. Biol. Chem. 254 8768, 8772 and 8777, 1979). Hayes and Castellino describe two plasminogen variants. Variant 1 contains an N-linked carbohydrate structure at $asn_{289}$ and an O-linked carbohydrate at $thr_{346}$. Variant 2 contains an O-linked carbohydrate at $thr_{346}$ but the N-linked carbohydrate at $asn_{289}$ is absent.

The glycosylation at an N-linked site depends upon the presence of a tripeptide recognition sequence asn-X-ser/thr
where X is other than proline.

According to the present invention there is provided plasminogen or a plasminogen activator containing the five kringle domains of plasminogen in which a carbohydrate structure cannot be attached to amino acid 289.

In a preferred aspect the plasminogen or plasminogen activator comprises an amino acid residue other than asparagine at position 289.

The replacement amino acids are chosen so as to avoid a side chain which is capable of participation in post-synthetic modification e.g. a residue capable of disulphide bond formation or a residue introducing a protease-sensitive cleavage site.

Suitable examples of residues to replace $asn_{289}$ include glutamine and aspartic acid.

In a preferred aspect, the plasminogen or plasminogen activator preferably also comprises an amino acid residue other than threonine at position 346, such as alanine or glycine. It will thus be appreciated that both potential plasminogen glycosylation sites are blocked.

Examples of plasminogen modified in accordance with the invention include:

Plg $asn_{289}$ -> gln; $thr_{346}$ -> ala
and Plg $asn_{289}$ -> asp

Plasminogen modified in accordance with the invention may be employed in plasminogen-containing plasminogen activators which also form part of the invention.

Suitable examples of plasminogen activators include streptokinase-plasminogen activator complex; acylated streptokinase-plasminogen activator complexes including those described in EP-0009879, EP-0028489 and EP-0091240, especially p-anisoyl streptokinase-plasminogen activator complex with substantially no internal peptide bond cleavage; and hybrid plasminogen activators (hybrid PA's) containing the five kringle domains of plasminogen, such as descibed in EP-0155387, EP-0290118 (USSN 163959), EP-0292326 (USSN 195207) and EP-0297882 (USSN 212910) (all incorporated herein by reference).

EP-0009879 discloses derivatives of in vivo fibrinolytic enzymes which are useful therapeutic agents for treating venous thrombosis. The derivatives are characterised by the active catalytic site on the enzymes being blocked by a group which is removable by hydrolysis such that the pseudo first order rate constant for hydrolysis is in the range $10^{-6}$ to $10^{-3}$ $sec^{-1}$.

EP-0028489 discloses, inter alia, p-anisoyl streptokinase-lys plasminogen activator complex with substantially no internal peptide bond cleavage. The present invention provides, in particular: p-anisoyl streptokinase-lys plasminogen ($asn_{289}$->gln; $thr_{346}$->ala) activator complex with substantially no internal peptide bond cleavage; p-anisoyl streptokinase-glu plasminogen ($asn_{289}$->gln; $thr_{346}$->ala) activator complex with substantially no internal peptide bond cleavage. These compounds may be prepared by the procedures generally described in EP-0028489 but using lys or glu plasminogen ($asn_{289}$->gln; $thr_{346}$->ala).

Specifically the process comprises mixing streptokinase with plasminogen according to the invention in the presence of an excess of a blocking agent of the formula EF where E is a locating group which locates the agent in the catalytic site and F is a group which is capable of transferring from the locating group to the catalytic site, such as p-amidinophenyl p'-anisate.

EP-0155387 discloses fibrinolytically active hybrid protein which comprises one chain of a 2-chain protease linked to a chain of a different 2-chain protease, or to the same chain of the same protease, at least one of the chains in the hybrid protein being derived from a fibrinolytically active protease, such that the hybrid protein has a catalytic site essential for fibrinolytic activity which is optionally blocked by a removable blocking group.

Examples of the hybrid protein include plasmin A-chain linked to tissue-type plasminogen activator (t-PA) B-chain or urokinase B-chain.

The hybrid proteins may be prepared by mixing a chain of one protease with a chain of another protease, optionally with dialysis, under oxidative conditions.

Alternatively, the hybrid proteins may be prepared by taking the genetic information (DNA sequence) of each protein, cutting and ligating this to construct a new DNA sequence coding for the hybrid protein, and expressing this DNA in prokaryote or eukaryote hosts.

The sequence of amino acids making up the enzyme tissue-type plasminogen activator (t-PA) and the nucleotide sequence for the cDNA which codes for t-PA are known (see Pennica et al., 1983; Nature, 301, 214). t-PA is known to have fibrinolytic activity.

As used herein, the term tissue-type plasminogen activator (t-PA) denotes a plasminogen activator of the group having the immunological properties defined for t-PA at the XXVIII Meeting of the International Committee on Thrombosis and Haemostasis, Bergamo, Italy, 27 July 1982.

The amino acid sequence of various forms of t-PA are known. The abovementioned Nature 1983 reference discloses the sequence for the L-chain and the mature S-chain forms of t-PA, also discussed by Vehar et al., Biotechnology, 1984, 2, 1051-7 in which the processing of initially formed t-PA by removal of a pro-sequence to give the S-chain form is reported. Pohl et al., FEBS letters, 1984, Vol. 168 No. 1, 29-32, refers to the N-terminal multiplicity of t-PA and discloses the U-chain form. The numbering system for the amino acid sequence of t-PA used herein is that described in the Nature 1983 reference for mature (S-chain) t-PA in which the N-terminal serine is numbered 1. By this system, L-chain t-PA has an N-terminal glycine residue at position -3 and U-chain t-PA has an N-terminal valine at position 4. References to t-PA herein are understood to include all such variant forms.

Native t-PA is composed of a B or light (L) and an A or heavy (H) chain. The B-chain contains the active site of the enzyme. The cleavage site for the conversion of t-PA from the single to the two-chain form is located between residues arg-275 and ile-276. In the two-chain form the chains are held together by a disulphide bridge formed between residues cys-264 in the A-chain and cys-395 in the B-chain.

It has been shown (Ny, T. et al, 1984; Proc. Natl. Acad. Sci. U.S.A., 81, 5355) that the A chain exhibits a number of structural and functional domains which are homologous to structures found in other plasma proteins: two triple disulphide-bonded structures or kringles, a growth-factor-like domain and a fibronectin-finger-like domain.

The region from amino acid residues 44 to 91 has been termed the "growth factor domain" (Banyai, L., et al FEBS Lett. 163, 37, 1983). The genetic information which codes for the major part of this domain, residues 51 to 86 inclusive, and partially codes for residues 50 and 87, lies on a single exon. The region from the first to last cysteine residues within this region, residues 51 to 84 inclusive, defines a triple-disulphide linked structure which will be referred to herein as the t-PA growth factor domain.

Fibronectin has twelve finger-domains per monomer, responsible for fibrin-affinity (Eur. J. Biochem. 154, 15-29 (1986)). The amino acid sequences of these finger domains are known (EMBO J.4, 1755 - 1759 (1985); Eur. J. Biochem. 128, 605-623 (1982); Proc. Natl. Acad. Sci. USA 80, 137-141 (1983)). It has been shown (J. Biol. Chem. 260, 5328-5341 and 13666-13676 (1985)) that part of Factor XII shows structural homology with the finger-domains of fibronectin. It has also been shown (Banyai, L. et al, 1983; FEBS Lett., 163, 37)that a part of the t-PA enzyme shows structural homology with the finger-domains of fibronectin. This region from amino acid residue 6 to 43 has been termed the t-PA finger domain. The genetic information for this domain lies on a single exon (Ny, T. et al, 1984; Proc. Natl. Acad. Sci. U.S.A., 81, 5355). The term "finger domain" will be used hereinafter to refer to an amino acid sequence showing structural homology with the finger-domains of fibronectin, or the sequence of a fibronectin finger-domain itself.

The sequence of amino acids making up the enzyme urokinase-type plasminogen activator (u-PA) in its single chain and two chain forms (Verstraete, M. and Collen, D., 1986; Blood, 67, 1529) and the nucleotide sequence for the cDNA which codes for human u-PA (Holmes, W. E. et al, 1985; Bio/technology 3, 923-929) are known. Urokinase-type plasminogen activator is known to have fibrinolytic activity. The two chains of u-PA are termed the A- and B-chain. The B-chain contains the active site of the enzyme. The cleavage site for the conversion of u-PA from the single to the two chain form is located between residues lys-158 and ile-159. In the two chain form the chains are held together by a disulphide bridge formed between residues cys-148 in the A-chain and cys-279 in the B-chain.

As used herein, the term urokinase-type plasminogen activator (u-PA) denotes a plasminogen activator of the group having the immunological properties defined for u-PA at the XXVIII Meeting of the International Committee on Thrombosis and Haemostasis, Bergamo, Italy, 27 July 1982.

The numbering system for the amino acid and nucleotide sequence of u-PA used herein is that described in Holmes, W. E. et al, 1985 (op. cit.) in which the N-terminal serine residue is numbered 1.

In addition to the native forms of t-PA and u-PA described above, various muteins are also known, see for example EP-A-0201153, EP-A-0233013, EP-A-0199574, WO 86/01538, EP-A-0227462, EP-A-0253582, WO 86/04351, EP-A-0236040, EP-A-0200451, EP-A-0238304, EP-0225286, DE 3537176, WO 87/04722 and EP-A-0236289.

References herein to t-PA and u-PA species include both native forms and muteins.

EP-0297882 (USSN 212910) discloses a hybrid plasminogen activator (hybrid PA) which comprises the five kringle domains of plasminogen linked to the B-chain of t-PA or u-PA via an amino acid sequence comprising, respectively, the t-PA cleavage site between residues 275 and 276 and the cysteine residue 264 of t-PA or the u-PA cleavage site between residues 158 and 159 and the cysteine residue 148 of u-PA.

It will be understood that by the term 'B-chain' is meant at least that portion of the B-chain containing the functional active centre of t-PA or u-PA, and preferably comprises residues 276-527 or 159-411 respectively.

The linking sequence of amino acids may be introduced synthetically during the preparation of the hybrid plasminogen activator (PA) and/or derived from native sequences.

Native plasminogen includes cysteine residues at positions 548 and 558, C-terminal to plasminogen kringle 5, which participate in the interchain disulphide bridges of the two-chain plasmin form. In the preferred embodiment these residues are not present in the linking sequence.

It will be appreciated that to prevent cleavage of the plasminogen kringles from the t-PA or u-PA B-chain in vivo, the linking sequence should be chosen so as to avoid the presence of a site susceptible to trypsin-like proteolytic cleavage N-terminal to residue cys-264 of t-PA or cys-148 of u-PA, as appropriate.

Where the B-chain of t-PA is employed, the linking sequence of amino acids preferably comprises t-PA residues 264 to 275 inclusive, more preferably residues 262 to 275 inclusive.

Where the B-chain of u-PA is employed, the linking sequence of amino acids preferably comprises u-PA residues 148 to 158 inclusive, more preferably residues 137 to 158 inclusive.

In one preferred aspect, the hybrid PA may be represented symbolically as:

$$(Y\text{-})_m(K^p\text{-}Z^t\text{-})_5 B^t$$

where $B^t$ comprises residues 276-527 of t-PA, m is 0 or 1, preferably 1, each of the 5 values of $K^p$ represents a kringle domain derived from plasminogen in sequence and Y and each of the 5 values of $Z^t$ independently represents a bond or a linking sequence of amino acids which may be introduced synthetically during the preparation of the hybrid PA and/or derived from native plasminogen and/or t-PA sequences, the sequence $Z_5^t$ comprising at least residues cys-264 and arg-275 of t-PA and the group $(K^p\text{-}Z^t)_5$ being modified in accordance with the invention.

In a second preferred aspect, the hybrid PA may be represented symbolically as:

$$(Y\text{-})_m(K^p\text{-}Z^u\text{-})_5 B^u$$

where $B^u$ comprises residues 159-411 of u-PA, Y and each of the 5 values of $Z^u$ independently represents a bond or a linking sequence of amino acids which may be introduced synthetically during the preparation of the hybrid PA and/or derived from native plasminogen and/or u-PA sequences, the sequence $Z_5^u$ comprising at least residues cys-148 and lys-158 of u-PA, m and each of the 5 values of $K^p$ are as previously defined and the group $(K^p-Z^u)_5$ being modified in accordance with the invention.

When m is 1, the sequence Y may take one of the values found in various forms of native plasminogen, such as the $glu_1$ or $lys_{78}$ forms, that is plasminogen residues 1 to 83 or 78 to 83 respectively.

Optionally, as taught generally in EP-A-0241210, the sequence Y may commence with one or more finger domains, defined herein as an amino acid sequence showing structural homology with the finger-domains of fibronectin, such as a fibronectin finger domain itself or a t-PA finger domain, preferably a t-PA finger domain.

Where a finger domain is derived from t-PA, the finger domain sequence may optionally extend at the N-terminus to include residues preceding residue 6 of native t-PA, such as residues 4 and 5, 1 to 5 or -3 to 5 respectively of the U-, S- or L-chain forms of native t-PA. The finger domain sequence preferably extends at the N-terminus to comprise residues 1 to 5 of native t-PA.

Each finger domain sequence may optionally be linked to a second sequence of amino acids which corresponds to the growth-factor domain of native t-PA. Thus, representing the finger domain sequence (for example residues 6 to 43 of t-PA) as F and the growth-factor domain sequence (residues 51 to 84 of t-PA) as G, Y comprises one or more N-terminal units of the form $A-F-X_1-$ and/or $A-F-X_2-G-X_3-$, where $X_1$, $X_2$ and $X_3$ represent bonds or linking sequences of amino acids which may be introduced synthetically during the preparation of the hybrid PA and/or be derived from native t-PA sequences adjacent to the F and G domains and A is an optional N-terminal extension of the F domain.

In a preferred aspect, the linking sequence $X_1$ comprises amino acid residues selected from the residues 44 to 50 and 85 to 91, and more preferably comprises residues 44 to 50 and 88 to 91, of native t-PA, optionally linked at the C-terminus to a sequence of amino acids, such as -pro-gly-. The linking sequence $X_2$ preferably comprises residues selected from the residues 44 to 50 of native t-PA, and more preferably comprises residues 44 to 50. The linking sequence $X_3$ preferably comprises residues selected from the residues 85 to 91 of native t-PA and more preferably comprises residues 85 to 91, optionally linked at the C-terminus to a sequence of amino acids such as -pro-gly-.

It will be appreciated that to prevent cleavage of the additional domain(s) from one another or from the remainder of the hybrid plasminogen activator in vivo, inter-domain linking sequences and linking sequences between the additional domain(s) and the plasminogen activator molecule should be chosen so as to avoid the presence of a site susceptible to proteolytic cleavage. Thus, in particular, the linking sequence $X_1$ or $X_3$ will preferably end with a residue other than arginine or lysine.

Y preferably comprises up to 6 additional finger domains, more preferably up to 2, most preferably 1, each of which may optionally be linked to a growth factor domain.

$Z_1$, $Z_2$, $Z_3$ and $Z_4$ preferably represent the native plasminogen inter-domain sequences between plasminogen kringle domains 1 and 2, 2 and 3, 3 and 4 and 4 and 5, respectively, $Z_3$ being optionally modified at $thr_{346}$ in accordance with the invention.

Suitable sequences $(Z_5^t)$ linking the C-terminal plasminogen kringle domain to the t-PA B-chain include:

1. [AAPSTCGLRQYSQPQFR]
2. [AAPSTCGLRQYSQPQFQ]
3. [STCGLRQYSQPQFR] (single letter amino acid notation) from which it can be seen that the sequences 1 and 2 consist of residues 542-544 of plasminogen and residues 263 to 275 of t-PA linked by a serine residue. The interposed serine residue can be identified with ser-545 of plasminogen or ser-262 of t-PA. In sequence 2, residue 275 of t-PA has been replaced by glutamine in accordance with EP-A-0233013. Sequence 3 consists of residues 262 to 275 of t-PA.

The preferred sequence $(Z_5^u)$ linking the C-terminal plasminogen kringle domain to the u-PA B-chain is:

[AAPSFPSSPPEELKFQCGQKTLRPRFK]

(single letter amino acid notation) from which it can be seen that the sequence consists of residues 542-546 of plasminogen and residues 137 to 158 of u-PA.

The preferred hybrid PA's of the invention have the following structures:

1. Plg 1-541($asn_{289}$->)gln)[AAPSTCGLRQYSQPQFR]t-PA 276-527
2. Plg 1-541($asn_{289}$->gln; $thr_{346}$->ala) [AAPSTCGLRQYSQPQFR]t-PA 276-527
3. Plg 1-541($asn_{289}$->)gln; $thr_{346}$->ala) [AAPSTCGLRQYSQPQFR]t-PA 276-527($asn_{448}$->gln)
4. Plg 1-541($asn_{289}$->gln; $thr_{346}$->ala) [STCGLRQYSQPQFR]t-PA 276-527($asn_{448}$->gln)
5. Deglyco plg 1-541[AAPSTCGLRQYSQPQFR]t-PA 276-527 (product of the treatment of the glycosylated compound with peptide:N-glycosidase F)
6. Deglyco plg 1-541[STCGLRQYSQPQFR]t-PA 276-527 (product of the treatment of the glycosylated compound with peptide:N-glycosidase F)

7. Deglyco plg 1-541[AAPSFPSSPPEELKFQCGQKTLRPRFK] u-PA 159-411 (product of the treatment of the glycosylated compound with peptide:N-glycosidase F), where Plg 1-541 represents residues 1-541 of plasminogen (that is, including kringles 1 to 5) and the symbols in brackets represent amino acid residues according to the single letter amino acid notation, including one and two chain variants, $lys_{78}$ and $glu_1$ variants, and mixtures thereof.

The plasminogen activator (PA) of the invention may be derivatised to provide pharmaceutically useful conjugates analogous to known PA-containing conjugates, for example:

(a) an enzyme-protein conjugate as disclosed in EP-A-O 155 388, in which the catalytic site on the enzyme which is responsible for fibrinolytic activity is blocked by a human protein attached thereto by way of a reversible linking group;

(b) an enzyme-protein conjugate as disclosed in EP-A-O 152 736, comprising at least one optionally blocked fibrinolytic enzyme linked by way of a site other than the catalytic site responsible for fibrinolytic activity to at least one human protein;

(c) a protein-polymer conjugate as disclosed in EP-A-0183503 comprising a pharmaceutically useful protein linked to at least one water soluble polymer by means of a reversible linking group; or

(d) an enzyme conjugate as disclosed in EP-A-0184363 comprising a plurality of fibrinolytic enzymes linked together through the active centres thereof by means of a removable blocking group.

The PA of the invention may take the place of a PA as the enzyme or (human) protein component, as appropriate, of any of the conjugates described above.

The above mentioned derivatives of the PA may be used in any of the methods and compositions described hereinafter for the PA itself.

In one further aspect, the invention provides a process for preparing plasminogen or plasminogen activator according to the invention which process comprises treating the corresponding protein having a carbohydrate structure at position $asn_{289}$ with peptide:N-glycosidase F.

Peptide:N-glycosidase F (T.H. Plummer, J.H. Elder, S. Alexander, A.W. Phelan and A.L. Tarentino, J. Biol. Chem. (1984) 259 (17) 10700-10704) is a carbohydrate chain-cleaving enzyme from Flavobacterium meningosepticum, commercially available under the Tradename Glycopeptidase F (Boehringer Mannheim), which is believed to hydrolyse a glycoprotein at the glycosylamine junction to release the carbohydrate moiety and convert the protein asparagine to aspartic acid at the point of cleavage.

In a second further aspect, the invention provides a process for preparing plasminogen or plasminogen activator according to the invention which process comprises expressing DNA encoding said protein in a recombinant host cell and recovering the protein product.

The DNA polymer comprising a nucleotide sequence that encodes the protein also forms part of the invention.

The process of the invention may be performed by conventional recombinant techniques such as described in Maniatis et. al., Molecular Cloning - A Laboratory Manual; Cold Spring Harbor, 1982 and DNA Cloning vols I, II and III (D.M. Glover ed., IRL Press Ltd).

In particular, the process may comprise the steps of:

i) preparing a replicable expression vector capable, in a host cell, of expressing a DNA polymer comprising a nucleotide sequence that encodes said protein.

ii) transforming a host cell with said vector;

iii) culturing said transformed host cell under conditions permitting expression of said DNA polymer to produce said protein; and

iv) recovering said protein.

The invention also provides a process for preparing the DNA polymer by the condensation of appropriate mono-, di- or oligomeric nucleotide units.

The preparation may be carried out chemically, enzymatically, or by a combination of the two methods, in vitro or in vivo as appropriate. Thus, the DNA polymer may be prepared by the enzymatic ligation of appropriate DNA fragments, by conventional methods such as those described by D. M. Roberts et al in Biochemistry 1985, 24, 5090-5098.

The DNA fragments may be obtained by digestion of DNA containing the required sequences of nucleotides with appropriate restriction enzymes, by chemical synthesis, by enzymatic polymerisation, or by a combination of these methods.

Digestion with restriction enzymes may be performed in an appropriate buffer at a temperature of 20°-70°C, generally in a volume of 50µl or less with 0.1-10µg DNA.

Enzymatic polymerisation of DNA may be carried out in vitro using a DNA polymerase such as DNA polymerase I (Klenow fragment) in an appropriate buffer containing the nucleoside triphosphates dATP, dCTP, dGTP and dTTP as required at a temperature of 10°-37°C, generally in a volume of 50µl or less.

Enzymatic ligation of DNA fragments may be carried out using a DNA ligase such as T4 DNA ligase in an appropriate buffer at a temperature of 4°C to ambient, generally in a volume of 50µl or less.

The chemical synthesis of the DNA polymer or fragments may be carried out by conventional phosphotriester, phosphite or phosphoramidite chemistry, using solid phase techniques such as those described in 'Chemical and Enzymatic Synthesis of Gene Fragments - A Laboratory Manual' (ed. H.G. Gassen and A. Lang), Verlag Chemie, Weinheim (1982),or in other scientific publications, for example M.J. Gait, H.W.D. Matthes, M. Singh, B.S. Sproat, and R.C. Titmas, Nucleic Acids Research, 1982, 10, 6243; B.S. Sproat and W. Bannwarth, Tetrahedron Letters, 1983, 24, 5771; M.D. Matteucci and M.H Caruthers, Tetrahedron Letters, 1980, 21, 719; M.D. Matteucci and M.H. Caruthers, Journal of the American Chemical Society, 1981, 103, 3185; S.P. Adams et al., Journal of the American Chemical Society,1983, 105, 661; N.D. Sinha, J. Biernat, J. McMannus, and H. Koester, Nucleic Acids Research, 1984, 12, 4539; and H.W.D. Matthes et al., EMBO Journal, 1984, 3, 801. Preferably an automated DNA synthesizer is employed.

The precise structure of the DNA fragments and the way in which they are obtained depends upon the structure of the desired protein. The design of a suitable strategy for the construction of the DNA molecule coding for the protein is a routine matter for the skilled worker in the art.

The expression of the DNA polymer encoding the protein in a recombinant host cell may be carried out by means of a replicable expression vector capable, in the host cell, of expressing the DNA polymer. The expression vector is novel and also forms part of the invention.

The replicable expression vector may be prepared in accordance with the invention, by cleaving a vector compatible with the host cell to provide a linear DNA segment having an intact replicon, and combining said linear segment with one or more DNA molecules which, together with said linear segment, encode the protein, under ligating conditions.

The ligation of the linear segment and more than one DNA molecule may be carried out simultaneously or sequentially as desired.

Thus, the DNA polymer may be preformed or formed during the construction of the vector, as desired.

The choice of vector will be determined in part by the host cell, which may be prokaryotic, such as E. coli, or eukaryotic, such as mouse C127, mouse myeloma, chinese hamster ovary, fungi e.g. filamentous fungi or unicellular 'yeast' or an insect cell such as Drosophila. The host cell may also be in a transgenic animal. Suitable vectors include plasmids, bacteriophages, cosmids and recombinant viruses, derived from, for example, baculoviruses or vaccinia.

The preparation of the replicable expression vector may be carried out conventionally with appropriate enzymes for restriction, polymerisation and ligation of the DNA, by procedures described in, for example, Maniatis et al., cited above. Polymerisation and ligation may be performed as described above for the preparation of the DNA polymer. Digestion with restriction enzymes may be performed in an appropriate buffer at a temperature of 20°-70°C, generally in a volume of 50µl or less with 0.1-10µg DNA.

The recombinant host cell is prepared, in accordance with the invention, by transforming a host cell with a replicable expression vector of the invention under transforming conditions. Suitable transforming conditions are conventional and are described in, for example, Maniatis et al., cited above, or "DNA Cloning" Vol. II, D.M. Glover ed., IRL Press Ltd, 1985.

The choice of transforming conditions is determined by the host cell. Thus, a bacterial host such as E. coli may be treated with a solution of CaCl₂ (Cohen et al, Proc. Nat. Acad. Sci., 1973, 69, 2110) or with a solution comprising a mixture of RbCl, MnCl₂, potassium acetate and glycerol, and then with 3-[N-morpholino]-propane-sulphonic acid, RbCl and glycerol. Mammalian cells in culture may be transformed by calcium co-precipitation of the vector DNA onto the cells. Where the host cell is in a transgenic animal, transformation may be effected by microinjection (see, for example, Gordon, K. et al, Biotechnology 1987 5 1183). The transformation of filamentous fungi has been reviewed (Timberlake, W.E. and Marshall, M.A., Science 1989 244 1313).

The invention also extends to a host cell transformed with a replicable expression vector of the invention. Culturing the transformed host cell under conditions permitting expression of the DNA polymer is carried out conventionally, as described in, for example, Maniatis et al and "DNA Cloning" cited above. Thus, preferably the cell is supplied with nutrient and cultured at a temperature below 45°C.

The protein expression product is recovered by conventional methods according to the host cell. Thus, where the host cell is bacterial, such as E. coli it may be lysed physically, chemically or enzymatically and the protein product isolated from the resulting lysate or a secretion vector may be used and the product isolated from the periplasm or from the nutrient medium. Where the host cell is mammalian, the product may generally be isolated from the nutrient medium. Where the host cell is in a transgenic animal, the product may be isolated from the milk.

The DNA polymer may be assembled into vectors designed for isolation of stable transformed mammalian cell lines expressing the protein; e.g. bovine papillomavirus vectors or amplified vectors in chinese hamster ovary cells (DNA cloning Vol.II D.M. Glover ed. IRL Press 1985; Kaufman, R.J. et al., Molecular and Cellular Biology 5, 1750-1759, 1985; Pavlakis G.N. and Hamer, D.H., Proceedings of the National Academy of Sciences (USA) 80, 397-401, 1983; Goeddel, D.V. et al., European Patent Application No. 0093619, 1983).

It will be appreciated that, depending upon the host cell, the protein prepared in accordance with the invention may be glycosylated to varying degrees. Furthermore, as observed by Pohl et.al., Biochemistry, 1984, 23, 3701-3707 and Rasmussen, J.R. et al., Biotech 88, p.97, Online Publications, Pinner, UK, 1988, varying degress of glycosylation may also be found in unmodified, naturally occurring t-PA. Plasminogen also exhibits varying degrees of glycosylation (Hayes M.L, J. Biol. Chem. 254: 8768, 1979 and Marti, J. et al., Eur. J. Biochem. 173, 57, 1988). Mutant forms of the protein product are also contemplated in which remaining glycosylation sites are removed by genetic engineering techniques, for example as taught in EP-A-0238304, EP-A-0225286, DE-3537176 or WO87/04722, such as where $asn_{448}$ in t-PA chain is converted to glutamine. The protein of the invention is understood to include such glycosylated variations.

It will also be appreciated that, depending upon the expression conditions, the protein prepared in accordance with the invention may exist in the single or two chain forms or mixtures thereof. The invention extends to all such forms.

In one aspect of the invention, the protein is a hybrid PA of the invention comprising the B-chain of native t-PA or u-PA linked to an A-chain comprising the five kringle domains derived from plasminogen and linking sequence of amino acids comprising residues 264 and 275 of t-PA or residues 158 and 148 of u-PA, respectively.

This hybrid PA A-chain may be employed as one chain of a fibrinolytically active hybrid protein such as disclosed in EP-0 155 387. The hybrid A-chain, DNA encoding the hybrid A-chain and a hybrid protein comprising the hybrid A-chain linked to the B-chain of a fibrinolytically active protease, the catalytic site of which is optionally blocked by a removable blocking group, all form part of the invention.

The hybrid A-chain may be prepared by separation from the B-chain thereof by mild reduction. Alternatively the hybrid A-chain may be prepared by expressing DNA coding therefor in a recombinant host cell and recovering the hybrid A-chain product. The hybrid protein comprising the hybrid A-chain linked to the B-chain of a fibrinolytically active protease may be prepared by (a) mixing said A- and B-chains under oxidative conditions; or (b) ligating DNA encoding said A-chain to DNA encoding said B-chain and expressing the ligated DNA in a prokaryote or eukaryote host; and thereafter optionally blocking the catalytic site of the hybrid protein with a removable blocking group. The oxidation and reduction conditions are as generally described in EP-A-0 155 387.

The resulting hybrid protein may be used in any of the methods and compositions described hereinafter for the hybrid PA itself.

The protein of the invention or conjugate thereof can be further derivatised such that any catalytic site essential for fibrinolytic activity is optionally blocked by a removable blocking group.

As used herein the expression 'removable blocking group' includes groups which are removable by hydrolysis at a rate such that the pseudo-first order rate constant for hydrolysis is in the range of $10^{-6}$ $sec^{-1}$ to $10^{-2}$ $sec^{-1}$, more preferably $10^{-5}$ $sec^{-1}$ to $10^{-3}$ $sec^{-1}$, most preferably $10^{-5}$ $sec^{-1}$ to $10^{-3}$ $sec^{-1}$ in isotonic aqueous media at pH 7.4 at 37°C.

Such blocking groups are described in European Patent No.0009879 and include acyl groups such as optionally substituted benzoyl or optionally substituted acryloyl.

Suitable optional substituents for benzoyl blocking groups include halogen, $C_{1-6}$ alkyl, $C_{1-6}$ alkoxy, $C_{1-6}$ alkanoyloxy, $C_{1-6}$ alkanoylamino, amino or p-guanidino.

Suitable optional substituents for acryloyl blocking groups include $C_{1-6}$ alkyl, furyl, phenyl or $C_{1-6}$ alkylphenyl.

In one preferred aspect, where the plasminogen activator comprises the serine protease domain of t-PA or u-PA, the removable blocking group is a 2-aminobenzoyl group substituted in the 3- or 4-position with a halogen atom and optionally further substituted with one or more weakly electron-withdrawing or electon-donating groups, wherein the pseudo first order rate constant for hydrolysis of the derivative is in the range $6.0 \times 10^{-5}$ to $4.0 \times 10^{-4}$ $sec^{-1}$ when measured in a buffer system consisting of 0.05M sodium phosphate, 0.1M sodium chloride, 0.01% v/v detergent comprising polyoxyethylenesorbitan monoleate having a molecular weight of approximately 1300, at pH 7.4 at 37°C.

Preferably the pseudo first order rate constant for hydrolysis of the derivative is in the range $6.0 \times 10^{-5}$ to $2.75 \times 10^{-4}$ $s^{-1}$, preferably $6.0 \times 10^{-5}$ to $2.5 \times 10^{-4}$ $s^{-1}$, more preferably $6.0 \times 10^{-5}$ to $2.0 \times 10^{-4}$ $s^{-1}$, still more preferably $6.0 \times 10^{-5}$ to $1.5 \times 10^{-4}$ $s^{-1}$ and most preferably $7.0 \times 10^{-5}$ to $1.5 \times 10^{-4}$ $s^{-1}$.

Preferably, the 2-aminobenzoyl group is substituted with a halogen atom in the 4-position.

Preferably, the halogen atom is fluorine, chlorine or bromine.

When the group is further substituted, preferred groups include $C_{1-6}$ alkyl, $C_{1-6}$ alkoxy and $C_{1-6}$ alkenyl substituents in the 3- or 5-positions of the ring.

Preferably, the blocking group is 4-fluoro-2-aminobenzoyl, 4-chloro-2-aminobenzoyl or 4-bromo-2-aminobenzoyl.

A suitable detergent for use in the buffer is the product having the tradename Tween 80.

A process for preparing a derivative of the invention, comprises reacting the plasminogen activator with a blocking agent JK in which J is a locating group which mediates binding of the agent to the catalytic site of the enzyme and K is a 2-aminobenzoyl group substituted in the 3- or 4-position with a halogen atom and optionally further substituted with one or more weakly electron-withdrawing or electron-donating groups.

Examples of group J include 4-amidinophenyl and 2-chloro-4-amidinophenyl.

Preferred agents JK are 4-amidinophenyl 2'-amino-4'-fluorobenzoate; 4-amidinophenyl 2'-amino-4'-chlorobenzoate and 4-amidinophenyl 2'-amino-4'-bromobenzoate.

Blocking agents JK as above defined are disclosed in EP-0297882 (USSN212910).

The blocking reactions are preferably carried out in aqueous media, at a pH in the range 7.0 to 8.5 and at temperatures in the range 0°C to 30°C. The preferred concentration range for the blocking agent is 0.01 to 2.0 millimolar, and the preferred enzyme concentration range is 1 to 500 micromolar.

In certain cases it is desirable to carry out the blocking reaction in the presence of enzyme solubilising or stabilising additives such as 1-arginine, 1-lysine or 6-aminohexanoic acid. The materials have been found to be compatible with the blocking agents.

The protein and derivatives of the invention are suitably administered in the form of a pharmaceutical composition.

Accordingly the present invention also provides a pharmaceutical composition comprising a protein or derivative of the invention in combination with a pharmaceutically acceptable carrier.

The compositions according to the invention may be formulated in accordance with routine procedures as pharmaceutical compositions adapted for intravenous administration to human beings.

Typically compositions for intravenous administration are solutions of the sterile enzyme in sterile isotonic aqueous buffer. Where necessary the composition may also include a solubilising agent to keep the protein or derivative in solution and a local anaesthetic such as lignocaine to ease pain at the site of injection. Generally, the protein or derivative will be supplied in unit dosage form for example as a dry powder or water free concentrate in a hermetically sealed container such as an ampoule or sachette indicating the quantity of protein in activity units. Where composition comprises a derivative of the invention or where the protein includes a removable blocking group, an indication of the time within which the free protein will be liberated may be given. Where the protein is to be administered by infusion, it will be dispensed with an infusion bottle containing sterile pharmaceutical grade 'Water for Injection' or saline. Where the protein is to be administered by injection, it is dispensed with an ampoule of sterile water for injection or saline. The injectable or infusable composition will be made up by mixing the ingredients prior to administration.

The quantity of material administered will depend upon the amount of fibrinolysis required and the speed with which it is required, the seriousness of the thromboembolic condition and position and size of the clot. The precise dose to be employed and mode of administration must per force in view of the nature of the complaint be decided according to the circumstances by the physician supervising treatment. However, in general, a patient being treated for a thrombus will generally receive a daily dose of from 0.01 to 10 mg/kg of body weight, such as 0.10 to 2.0mg/kg, either by injection in for example up to five doses or by infusion.

Within the above indicated dosage range, no adverse toxicological effects are indicated with the compounds of the invention.

Accordingly, in a further aspect of the invention there is provided a method of treating thrombotic diseases, which comprises administering to the sufferer an effective non-toxic amount of protein or a derivative of the invention.

In another aspect the invention provides the use of a protein or a derivative of the invention for the manufacture of a medicament for the treatment of thrombotic diseases.

The invention also provides a protein or a derivative of the invention for use as an active therapeutic substance and in particular for use in the treatment of thrombotic diseases.

The following Methods and Examples illustrate the invention.

I. General Methods used in Examples

(i) DNA cleavage

In general the cleavage of about 1μg of plasmid DNA or DNA fragments was effected using about 5 units of a restriction enzyme (or enzymes) in about 20μl of an appropriate buffer solution.

(ii) Generation of blunt ends:

If blunt ends were required they were produced by treating the DNA preparation with DNA Polymerase I, Klenow fragment as described by Maniatis et al, (Molecular Cloning-A Laboratory Manual, Cold Spring Harbor Laboratory, 1982).

(iii) Generation of 'Sticky ends' using linkers:

Short kinased oligonucleotide linkers encoding single or multiple restriction sites were ligated onto blunt ended fragments, the linker(s) was digested with the appropriate restriction endonuclease producing the required 'sticky end' necessary for further manipulation. (Molecular Cloning-A Laboratory Manual, Cold Spring Harbor Laboratory, 1982)

(iv) Ligation of DNA Fragments:

Ligation reactions were carried out as described in Maniatis et al, (Molecular Cloning - A Laboratory Manual, Cold Spring Harbor Laboratory, 1982).

(v) Transformation

of plasmid DNA into E.coli used competent HB101 cells supplied by Gibco BRL (Paisley, Scotland), according to the manufacturers instructions.

(vi) Plasmid preparation:

Large scale preparation of plasmid DNA and plasmid mini-preparations were carried out as described in Maniatis et al, (Molecular Cloning - A Laboratory Manual, Cold Spring Harbor Laboratory, (1982)).

(vii) Isolation of DNA fragments from low-melting-point (LMP) agarose gels:

DNA fragments were isolated from LMP agarose gels as described by Maniatis et al, (Molecular Cloning-A Laboratory Manual, Cold Spring Harbor Laboratory, 1982). Alternatively the excised gel band was purified using GENECLEAN TM, (Stratech Scientific, London) used according to the manufacturers instructions.

(viii) Radioactive labelling of DNA fragments:

DNA fragments were radioactively labelled with [α-32P]dCTP according to the method of Feinberg and Vogelstein. The procedure was carried out by utilising an OligolabellingTMKit. (Pharmacia, Pharmacia AB, Molecular Biology Division, Uppsala, Sweden).

(ix) Oligonucleotides:

Oligonucleotides were made on an Applied Biosystems 381A DNA Synthesizer according to the manufacturers instructions and were kinased as described in Maniatis et al, op. cit. When used in plasmid construction the oligonucleotides were annealed by heating together at 95oC (unless otherwise specified) for 5 minutes and cooling slowly to room temperature. The annealed oligonucleotides were then ready for ligation. If four oligonucleotides were to be annealed the annealing reaction was followed as described above but was carried out as 2 reactions each containing a pair of complementary oligonucleotides. After cooling the 2 reactions were mixed prior to ligation.

(x) DNA sequencing

(a) Single-strand method

Nucleotide sequence determination was carried out by the Sanger dideoxy chain termination method as described in Sanger, S., Nicklen, S. and Coulson, A.R. (1977) Proc. Natl. Acad. Sci. USA 74, 5463-5467 using 35S-labelled dATP. This method was mainly used for sequencing long stretches of DNA

(b) Double-strand method

Sequencing was carried out using 'SequenaseTM' (United States Biochemical Corporation) essentially according to the manufacturers instructions. This method was mainly used for verification of oligonucleotide and oligonucleotide/plasmid junction sequences.

(xi) Transient expression of plasminogen activators or plasminogen from HeLa cells

(a) Small-scale

Cell preparation: cells were trypsinised and plated out at approx. $2.4 \times 10^5$ cells per 35mm dish and incubated in 1.5ml growth medium (this is Hepes buffered RPM1 1640 medium (041-2400) containing 10% Serum (021-6010), 1% stock solution of penicillin/streptomycin (043-5070), 2% sodium bicarbonate solution (043-5080), 1% stock Glutamine (043-5030); Gibco, Paisley, Scotland) at 37°C in a humidified incubator in an atmosphere of 5% $CO_2$/95% air. After 72h the cells were refed, and used for transfection 24h later.
Transfection procedure: Cultures were changed to Eagles MEM (041-1095), 10% serum, 1% penicillin/streptomycin, 1% Glutamine and 1% non-essential amino acids (043-1140) 3h before transfection. The transfections used calcium coprecipitation as described in 'DNA Cloning' Ed. D.M. Glover (Chap. 15, C. Gorman). Glycerol shock and 5mM butyrate treatments were used. Plasminogen activator(s) secreted by transfected cells was harvested in 1.0ml RPMI 1640 medium (as above but serum-free, containing 4% soybean peptone (Sigma)) for 24h and activity was measured using fibrin plates with reference to normal t-PA (J.H. Robinson, Thromb. Res., 1984; 33, 155).
Alternatively the HeLa cells were plated at $5 \times 10^5$ per dish, in which case the cells were refed after only 24h and then used as above.

(b) Large-scale

Cell preparation: cells were trypsinised and plated out at a density of approx. $2.5 \times 10^6$ cells per 175cm² flask in 30ml growth medium (above). After 72h an extra 25ml of growth medium was added and the cells were used for DNA transfection 24h later (as above). 25ml of harvest medium were used per flask.
Alternatively the cells were plated at a density of approximately $2.0 \times 10^6$ cells per flask and 25ml of growth medium were added after 96h incubation and the cells used as above.
The two seeding rates and feed times used in the small and large-scale protocols were designed to allow convenient timing of experiments. Both sets of protocols allowed efficient expression of activator(s).

(xii) Western blotting/Immunogold silver staining

Following SDS PAGE, proteins were blotted onto nitrocellulose (NC) by electrophoretic transfer overnight at 4°C,40V, 100mA in 25mM Tris/192mM Glycine/20% (v/v) Methanol pH 8.3. Unreacted sites on the NC were blocked with 5% bovine serum albumin (BSA)/phosphate-buffered saline (PBS) pH 7.2 for 1h, followed by a 2h incubation with rabbit anti-t-PA B chain IgG (48μg/ml) in 1% BSA/0.5% Tween 80/PBS pH 7.2 (diluent). The NC was washed, 3 x 5 mins, in 0.5% Tween 80/PBS pH 7.2 and then incubated for 1h in biotinylated secondary antibody (Janssen; 2μg/ml in diluent). The NC was washed as above and incubated in Auroprobe™ BL plus streptavidin (Janssen) 1:100 in diluent for 2h. The NC was washed as above, followed by 1x1 min in deionised water, and silver enhanced using IntenSE™ II kit (Janssen) for 15-20 min. All steps were carried out at ambient temperature with gentle shaking.

In some instances the primary antibody was changed to a murine monoclonal antibody against plasminogen (Product 3642, American Diagnostica); if this were the case the secondary antibody was changed also, to one directed at murine rather than rabbit IgG.

(xiii) Fibrin plate assay for plasminogen/streptokinase complex(s)

The fibrin plates contained bovine fibrinogen (4mg/ml), with endogeneous plasminogen, bovine thrombin (4NIHu/ml) and streptokinase (30nM). 10μl samples of medium or standard were applied in duplicate to fibrin plates and incubated at 37°C for 16h. Essentially linear calibration lines could be obtained by plotting log plasminogen/streptokinase concentration against lysis zone diameter.

(xiv) Streptokinase/S2251 assay

Plasminogen was measured in a chromogenic substrate assay, calibrated with glu-plasminogen (0.3 to 30nM) in control, unused harvest medium or buffer as appropriate.

25μl standard or test sample was mixed with 250μl 1mM H-D-val-leu-lys. p-nitroanilide.2HCl (S2251; Flow Laboratories) in 0.1M triethanolamine pH 8.0 containing 0.2mg ml$^{-1}$ soybean trypsin inhibitor with or without streptokinase (3μM). Solutions were incubated at 37°C for 1 to 24h depending on the level of activity present. $A_{405}$ values were determined using a Dynatech Minireader II microtitre plate reader.

II. Identification of nucleotides, amino acid residues, N-termini, protein domains and chain nature in the examples

(i) Sequences

Plasminogen amino acid numbering based on Sottrup-Jensen et al (1978) Atlas of Protein Sequence and Structure Vol. 5, Suppl. 3, p91, National Biomedical Research Foundation, Silver Spring, MD., but updated to include the extra amino acid residue identified by Forsgren, M. et al (1987) FEBS Letters, 213, 254-260. Plasminogen nucleotide sequences as in Forsgren et al. (op.cit.). All t-PA numbering as in Pennica et al (1983) op. cit. Unless otherwise indicated the nucleotide numbers refer to plasminogen.

(ii) N-termini

glu$_1$ indicates the protein is believed to comprise the native (mature) plasminogen N terminus i.e. amino acid residues 1 onwards.

lys$_{78}$ indicates the protein is believed to comprise the processed lys$_{78}$ N terminus or the alternative process forms at met$_{69}$ or val$_{79}$.

(iii) Protein Domains

The protein domains described in the examples have been abbreviated for convenience and are defined as follows:

1. t-PA: $B^t$ = amino acid residues 276 to 527 inclusive
2. plasminogen:

$K_1P$ = amino acid residues 84 to 162 inclusive
$K_2P$ = amino acid residues 166 to 243 inclusive
$K_3P$ = amino acid residues 256 to 333 inclusive
$K_4P$ = amino acid residues 358 to 435 inclusive
$K_5P$ = amino acid residues 462 to 541 inclusive
the 5 plasminogen kringles $K_1$-$K_5$ = amino acid residues 84 to 541 inclusive

(iv) Chain nature

sc, indicates that the protein is in single chain form.
tc, indicates that the protein is in two chain form.

(v) Vectors

pTRE12 -(EP-0201153)-basic expression vector
pTRE15 -(EP-0201153)-encodes wild-type t-PA

III. Construction and expression of hybrid proteins

The expression of the proteins of Reference Examples 1 and 2 and Examples 1 to 12 where appropriate was carried out in HeLa cells.

Reference Example 1

Plasminogen 1-544/t-PA 262-527 (H204)

1.1 Construction of a plasmid carrying plasminogen A-chain cDNA

A partial plasminogen cDNA clone was obtained, as two overlapping isolates, from a human liver cDNA library after screening with an oligonucleotide probe of sequence (A):
5′ CC CCT CAC ACA CAT AAC AGG 3′    (A)
corresponding to nucleotides 49 to 68 of the sequence described in Malinowski et al Biochemistry 23, p4243 (1984).

The DNA sequence of the portion of the plasminogen cDNA clone coding for the A-chain of the protein was determined and two differences to the plasminogen cDNA sequence of Forsgren et al (1987) were noted.

The nucleotide at position 846 is T, the nucleotide at position 948 is G.

The 5′ end of the cDNA was shown to correspond to nucleotide 27 of the sequence of Forsgren et al - (1987) and the cDNA extended to nucleotide 1865 in the 3′ direction.

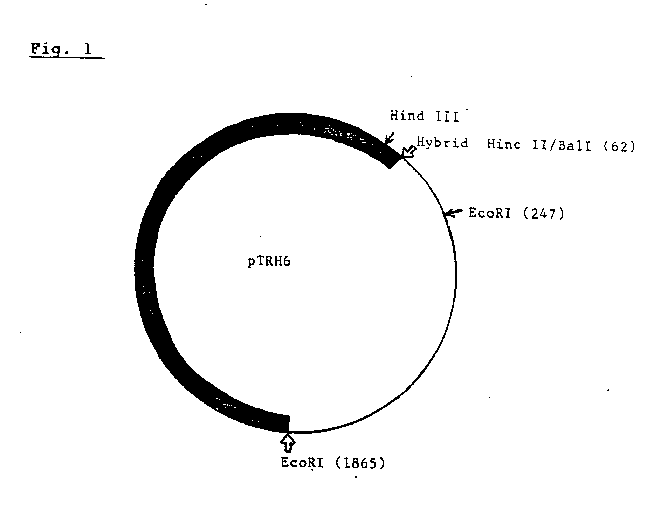

The partial plasminogen cDNA was subcloned into the vector pUC8 (Vieira and Messing, 1982, Gene 19, 259) between the HincII and EcoRI sites to create plasmid pTRH6 Fig. 1.

1.2 Modification of pTRE12

The single NarI site formerly in plasmid pTRE12 (EP-A-0 201 153) was destroyed by restriction cutting, blunt ending and religation forming pTRE12D Nar. Removal of the NarI site aided further manipulation.

1.3 Construction of a hybrid plasminogen: t-PA cDNA molecule (plasmid pTRH204)

Three fragments were prepared by restriction digestion and agarose gel electrophoresis. These fragments are as follows:-

I: approximately 1.6kb: from HindIII plus AhaII digest (HindIII [5′ to the plasminogen coding sequence] to AhaII(1572)) of pTRH6.

II: approximately 0.18kb: from an AhaII plus DdeI digest (AhaII (1572) to DdeI (1748)) of pTRH6.

III: approximately 5.3kb: largest fragment from a HindIII plus BgIII digest, of pTRE12D Nar. This fragment carries pTRE12D Nar vector functions.

These fragments (I, II, III) were ligated with a kinased oligonucleotide linker (linker IV) comprising two oligonucleotides of sequence:-

5′ TCAGTGTGCGGCGCCTGGTACCA 3′   (B)

5′ GATCTGGTACCAGGCGCCGCACAC 3′   (C)

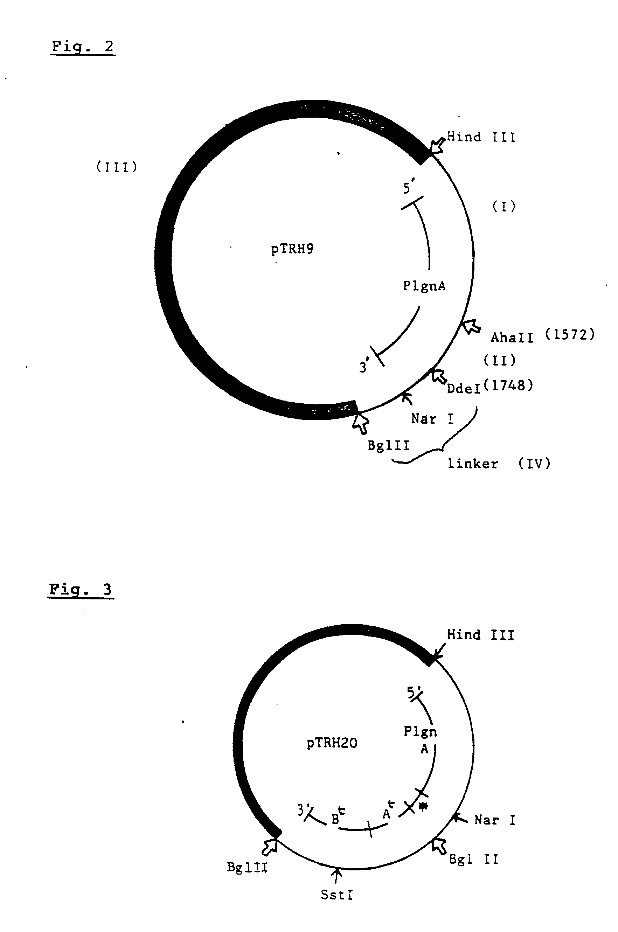

After transformation into E.coli HB101 the plasmid pTRH9 (Fig. 2) was isolated.

The DNA in plasmid pTRH9 encodes most of the plasminogen-A chain, up to and including proline 544 i.e. kringles 1 to 5; the DNA encoding the plasminogen B-chain and the remainder of the A-chain were absent. Use of linker IV in construction of pTRH9 introduces a unique NarI site, as may be seen from Table 1 (which shows the junction of fragments II and III and linker IV).

### TABLE 1

### Junction of fragments II and III and linker IV in plasmid pTRH9.

```
  Fragment              Linker                Fragment
     II                   IV                     III
 ────────────⇒⇐──────────────────────────⇒⇐─────────
        DdeI                   NarI              BglII

       1745      ⌒  1755      ⌒    1765        ⌒
5'... GAT GTC CCT CAG TGT GCG GCG* CCT GGT ACC AGA TCT
3'... CTA CAG GGA GTC ACA CGC CGC GGA CCA TGG TCT AGA

       asp val pro gln cys ala ala pro
       537 538 539 540 541 542 543 544
```

The plasminogen amino-acid sequence is shown

*This residue is C in plasminogen cDNA.

Nucleotide numbering is included (above the sequence).

Plasmid pTRH9 was restricted with BgIII and ligated with the 2kb BgIII fragment encoding the mature t-PA polypeptide isolated from pTRE15 (EP-A-0 201 153).

An E.coli HB101 clone carrying the t-PA insert in the same orientation (5′->3′) as the plasminogen 'A' chain DNA was isolated; this plasmid was called pTRH20 (see Fig. 3).

Plasmid pTRH20 encodes a molecule comprising the five kringles of plasminogen (residues 1 to 544) linked to the N-terminus of mature t-PA via the tripeptide gly-thr-arg. The junction of the plasminogen A-chain DNA and t-PA A-chain DNA of plasmid pTRH20 is shown in Table 2.

14

## TABLE 2

### Junction of the plasminogen A-chain DNA and t-PA A-chain in plasmid pTRH20.

```
                                        BglII

5'... CCT CAG TGT GCG GCG CCT GGT ACC AGA TCT TAC CAA .
3'... GGA GTC ACA CGC CGC GGA CCA TGG TCT AGA ATG GTT .
      pro gln cys ala ala pro gly thr arg ser tyr gln
      539 540 541 542 543 544             1   2   3


      Plasminogen        linking        t-PA
      sequences          tripeptide     sequences
```

The DNA encoding the t-PA A-chain and part of the B-chain was removed from pTRH20 by restriction with NarI and SstI (located at t-PA nucleotide 1417 as described by Pennica et al 1983). The large SstI -NarI fragment (7.9kb) was isolated (fragment V).

An approximately 0.4kb BanII fragment (fragment VI) was isolated from pTRE15. The two relevant BanII sites are located at nucletotides 1024 and 1417 in the t-PA cDNA sequence described by Pennica et al - (1983). BanII has a degenerate recognition site i.e.:

```
5' .... G Pu GC Py C.....3'
3' .... C Py CG Pu G.....5'
```

Note that the BanII site at 1417 is identical to the SstI site at 1417 and also that the sticky end created is compatible with an SstI sticky end. The 0.4kb BanII fragment encodes most of t-PA B-chain.

Two oligonucleotides (D) and (E):

```
5' CGCCGTCGACCTGCGGCCTGAGACAGTACAGCCAGCCTCAGTTTCGCATC

   AAAGGAGGGCT   3'                                    (D)


5' CTCCTTTGATGCGAAACTGAGGCTGGCTGTACTGTCTCAGGCCGCAGGTC

   GACGG  3'                                           (E)
```

were synthesized, kinased and combined to form linker VII.

Linker VII has a 5' NarI sticky end, compatible with the NarI sticky end of fragment V and a 3' BanII sticky end compatible with the BanII (nucleotide 1024) sticky end of fragment VI.

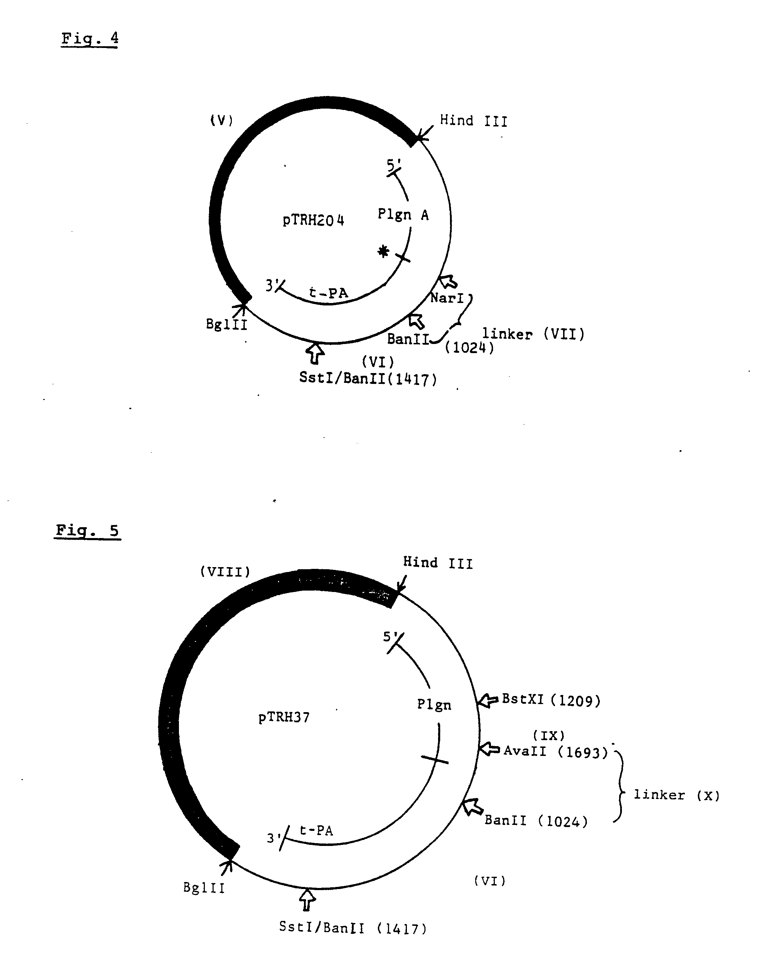

Fragments V and VI together with linker VII were ligated and transformed into E.coli HB101. A plasmid (pTRH204) with the structure depicted in Fig. 4 was isolated.

The junction of fragment V and linker VII is shown in Table 3.

Joining of fragments V and VI via the BanII (1417) and ssti sticky ends completed the reconstruction of the t-PA B-chain coding region.

## TABLE 3

## Junction of fragment V and linker VII in plasmid pTRH204

```
        Fragment V              linker VII
                                  NarI

   5'...CCT CAG TGT GCG GCG CCG TCG ACC TGC GGC CTG ...3'
   3'...GGA GTC ACA CGC CGC GGC AGC TGG ACG CCG GAC ...5'
        pro gln cys ala ala pro ser*thr cys gly leu
        539 540 541 542 543 544     263 264 265 266


              plasminogen                  t-PA
              sequences                    sequences
```

The plasminogen and t-PA amino-acid sequences at the junction are shown (plasminogen numbering 539-544 and t-PA numbering 263-266). The serine residue denoted * may be considered to be equivalent to plasminogen serine 545 or t-PA serine 262.

Plasmid pTRH204 was used to transfect human HeLa cells (as described in Methods) and produced a plasminogen activator (H204) with the structure: plasminogen 1-544/t-PA 262-527.

### 1.4 Purification of plasminogen 1-544/t-PA 262-527 (H204)

200ml conditioned harvest media obtained from HeLa cell cultures transfected with the plasmid pTRH204 (Reference Example 1.3) was centrifuged at 9000g for 30 min and the supernatant retained. It was applied to a column (i.d., 90 mm; h, 220 mm) of Sephadex G25 (Sephadex and Sepharose are Trade Marks) equilibrated in PBS (Dulbecco A)/0.01% Tween 80 pH 7.4 (PBS/TW). The first approx. 0.35 Vt was discarded. The following 500ml was retained and was chromatographed on zinc chelate-Sepharose (Vt = 80ml) and lysine Sepharose (Vt = 20ml) essentially as described previously (Dodd, I. et al (1986a) FEBS Lett 209 13). The major difference was that the purified activator was dissociated from the second column using 0.05M sodium phosphate/0.1M sodium chloride/0.01% Tween 80 pH 7.4 (PST) containing 0.02M E-aminocaproic acid ($\epsilon$-ACA). Active fractions eluted by the PST/$\epsilon$-ACA buffer were identified using the chromogenic substrate H-D-ile-pro-arg-p nitroanilide (Dodd, I. et al (1986b) Thromb. Haem. 55 94) and were concentrated by stirred-cell ultrafiltration (YM10, Amicon). The ultrafiltered retentate (2.0ml) contained 14400 IU and 4800 SU (Dodd, I. et al (1986b). Sodium dodecyl sulphate polyacrylamide gel electrophoresis (SDS PAGE) followed by fibrin zymography (Dodd, I. et al, 1986b) showed that the product contained a major fibrinolytic species with apparent Mr approximately 100000, consistent with the isolation of a glu$_1$ form of the hybrid activator.

### 1.5 Synthesis of lys$_{78}$ tc H204 protein (two chain plasminogen 78-544/t-PA 262-527)

A preparation of H204 protein (purified as described in Example 1.4) that had been buffer-exchanged into PST/10mg ml$^{-1}$ mannitol/50$\mu$M $\epsilon$-ACA (89,000 IU, 1.9 ml) was treated with a 0.01 fold molar excess of plasmin at 25°C for 16h and then stored at -40°C. Subsequent analysis of the product showed that it contained 68,000 IU and 32,000 SU. The activator had an apparent Mr of approximately 90,000 by SDS PAGE followed by fibrin zymography. The lys,tc nature of the product was confirmed by SDS PAGE followed by immuno blotting using a polyclonal antibody against the t-PA B-chain (see Methods section).


Reference Example 2


Plasminogen 1-541/t-PA 262-527(H37)


2.1 Construction of plasmid pTRH37

Plasmid pTRH37 was constructed by ligating 2 fragments from pTRH204 (Reference Example 1) together with fragment VI from pTRE15 and an oligonucleotide linker (linker X):

Fragment VIII : 7.2kb fragment of pTRH204 from the SstI site in the t-PA B-chain coding region (nucleotide 1417 in the t-PA sequence) to the BStXI site in the plasminogen A-chain coding region (nucleotide 1209 in the plasminogen sequence) (Fragment VIII thus contains all the vector sequences).

Fragment IX : 0.48Kb fragment of pTRH204 from the BstXI site (1209) to the AvaII site in the plasminogen A-chain coding region nucleotide (1693).

Fragment VI : (SstI-BanII) has been described previously (Reference Example 1).

Linker X was formed by annealing 4 oligonucleotides (F,G,H and I) of sequences:

```
5' GTCCCTGGTG CTACACGACA AATCCGCGGA AACTTTACGA
CTACTGTGAT GTCCCTCAGT GTTCGACCTG CGGCC 3'        (F)


5' TGAGACAGTA CAGCCAGCCT CAGTTTCGCA TCAAAGGAGG GCT 3'
                                                    (G)


5' CTCCTTTGAT GCGAAACTGA GGCTGGCTGT ACTGTCTCAG
GCCGCAGGTC GAACACTGAGGG 3'                       (H)


5' ACATCACAGT AGTCGTAAAG TTTCCGCGGA TTTGTCGTGT
AGCACCAGG 3'                                     (I)
```

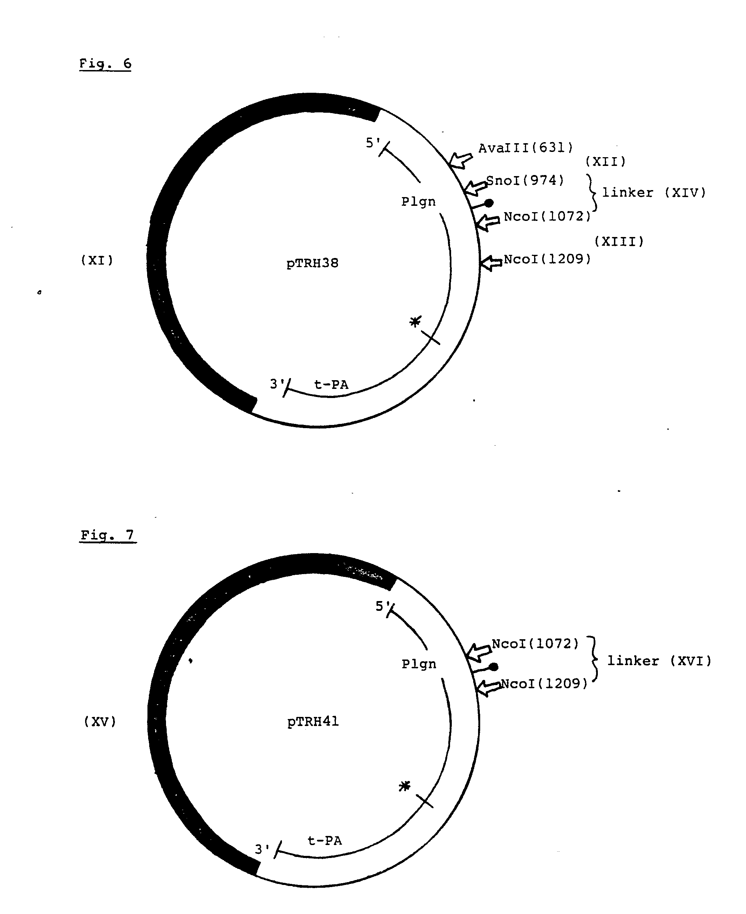

A plasmid (pTRH37) was isolated which has the structure shown in Fig. 5. The plasmid, when introduced into human HeLa cells, directed the expression of a novel plasminogen activator (H37) which has the same amino-acid sequence as protein H204 (Reference Example 1) except for a deletion of the amino-acid residues alanine (542), alanine (543) and proline (544) immediately C-terminal to the Kringle 5 domain. The hybrid protein structure between Kringle 5 and the protease cleavage site is shown below using single letter notation:

$$541* \qquad\qquad 276$$
$$\underline{C}STCGLRQYSQPQFR\underline{I}KGG$$
$$\uparrow$$

Residue 541 is the last cysteine in Kringle 5. The serine marked with an asterisk can be identified with serine545 of plasminogen or serine262 of t-PA.


2.2 Purification of plasminogen 1-541/t-PA 262-527 (H37)

510ml clarified, conditioned harvest medium that had been obtained from HeLa cell cultures that had been transfected with the plasmid pTR H37 (Reference Example 2.1) was purified essentially as described for H204 in Reference Example 1.4. The hybrid activator was dissociated from the lysine Sepharose column using PST/20mM $\epsilon$-ACA, was concentrated by ultrafiltration and was then buffer-exchanged into PST/10mg ml$^{-1}$ mannitol/50$\mu$M $\epsilon$-ACA (2.0ml). The product contained 36,000 IU and 15,000 SU. Analysis by SDS PAGE followed by fibrin zymography showed the preparation contained two major fibrinolytic species, at apparent Mr of approximately 100,000 and 90,000. These are believed to be the glu1 and lys78 forms of the activator. SDS PAGE followed by silver staining of non-reduced activator showed the preparation contained major protein bands at Mr approximately 90,000.

Analysis by SDS PAGE followed by immunoblotting using a polyclonal antibody against the t-PA B chain supported the zymography results indicating the presence of $glu_1$ and $lys_{78}$ forms, and, in addition, showed that the product was all two-chain.

## Example 1

### Deglyco plasminogen 78-544/t-PA 262-527 two chain

Purified $lys_{78}$ to H204 protein (400 pmoles, 150$\mu$l), prepared as described in Reference Example 1 was treated with Glycopeptidase F (Glycopeptidase F is a Trade Mark.) (7U, 36$\mu$l, Boehringer Mannheim) in a total incubation volume of 260$\mu$l in 25mM NaH$_2$PO$_4$/25mM EDTA/0.5% Triton X-100 pH 7.5 at 37°C for 24h. The product showed a similar dose response on fibrin plates to the native glycoprotein and was characterised as a deglycosylated $lys_{78}$ tc H204 protein because

1) by SDS PAGE followed by fibrin zymography there was a small decrease in the apparent M$_r$ of the Glycopeptidase F-treated $lys_{78}$ tc H204 protein compared to the native material,

2) by SDS PAGE under reducing conditions followed by Western blotting/Immunogold silver staining (see Methods Section) there was a small decrease in the Mr of the t-PA B chain moiety of Glycopeptidase F-treated $lys_{78}$ tc H204 protein.

## Example 2

### Synthesis of deglyco plasminogen 1-544/t-PA 262-527

A preparation of sc H204 protein (0.2 nmoles, 80$\mu$l), purified as described in Reference Example 1, was treated with Glycopeptidase F™ (2.9U, 14.4ul, Boehringer Mannheim) in a total incubation volume of 100$\mu$l in 25mM NaH$_2$PO$_4$/25mM EDTA/0.5% Triton X-100 pH 7.5 at 37°C for 24h. The product showed a similar dose response on fibrin plates to the native glycoprotein and was characterised as a deglycosylated sc H204 protein by the methods describedin Example 1.

## Example 3

### Plasminogen 1-544(asn$_{289}$->gln)/t-PA 262-527(H38)

#### 3.1 Construction of plasmid pTRH38 and expression of H38 protein

Plasmid pTRH38 was constructed by ligating 3 DNA fragments derived from pTRH204 (Reference Example 1) together with an oligonucleotide linker (linker XIV).
Fragment XI : 7.6kb fragment of pTRH204 from the NcoI site in plasminogen at 1209 to the AvaIII site in plasminogen at 631. This fragment therefore contains all the vector sequences.
Fragment XII : 0.34kb AvaIII (631) to SnoI (974) fragment from pTRH204.
Fragment XIII : 0.14kb NcoI fragment from pTRH204 between the sites at 1072 and 1209 in plasminogen.
Linker XIV : was formed by annealing 2 oligonucleotides (J and K):

18

5' TGCACAGACC CCTCACACAC ATCAGAGGAC ACCAGAAAAC

TTCCCCTGCA AAAATTTGGA TGAAAACTAC TGCCGCAATC CTGACGGAAA

AAGGGCCC 3'  (J)

5' CATGGGGCCC TTTTTCCGTC AGGATTGCGG CAGTAGTTTT

CATCCAAATT TTTGCAGGGG AAGTTTTCTG GTGTCCTCTG ATGTGTGTGA

GGGGTCTG 3'  (K)

A plasmid (pTRH38) with the structure depicted in Fig. 6 was isolated. When human HeLa cells were transfected with plasmid pTRH38 a novel plasminogen activator (H38) was produced which has the same structure as H204 except that the N-linked glycosylation site at $asn_{289}$ in the plasminogen A chain portion of the molecule is replaced with a glutamine residue. The protein is not, therefore, glycosylated at this site.

### 3.2 Purification of plasminogen 1-544($asn_{289}$->gln)/t-PA 262-527 (H38)

380 ml conditioned harvest media (see Methods) obtained from HeLa cell cultures transfected with the plasmid pTRH38 was centrifuged at 9000g for 30 min and the supernatant retained. It was applied to a column (i.d., 90 mm; h., 257 mm) of Sephadex G25 that had been equilibrated in PBS (Dulbecco A)/0.01% Tween 80 pH 7.4 (PBS/TW). The first approx. 0.35 Vt was discarded. The following 560 ml were retained. This fraction contained the buffer-exchanged H38. It was then chromatographed on Zinc chelate Sepharose CL4B (Vt. 80 ml) and lysine Sepharose CL4B (Vt, 40 ml) essentially as described previously (Dodd, I. et al 1986a FEBS Lett. 209 13). The major difference was that the H38 protein was dissociated from the lysine Sepharose column using 0.05M sodium phosphate/0.1M sodium chloride/0.01% Tween 80 (PST) containing 0.02M $\epsilon$-aminocaproic acid. ($\epsilon$-ACA). Fractions containing the purified H38 protein were identified by chromogenic substrate assay using S2288 (Dodd, I. et al 1986b Thromb. Haemostas. 55 94), were pooled and were concentrated by stirred cell ultrafiltration using a membrane (YM10, Amicon) with a molecular weight cut-off of 10,000. The ultrafiltered retentate was buffer-exchanged into PST/10 mg ml$^{-1}$ mannitol/50$\mu$M $\epsilon$-ACA pH 7.4 (2.5 ml) using Sephadex G25 (PD10, Pharmacia). This G25 eluate was regarded as the purified product.

The purified product contained 29000 IU/ml and 11,500 SU/ml (Dodd, I. 1986b). Analysis by SDS PAGE followed by fibrin zymography (Dodd, I. 1986b) revealed two species, one with apparent $M_r$ 100,000, and a minor species with apparent $M_r$ 90,000. These are believed to be the $glu_1$ and $lys_{78}$ forms of protein H38. The material was all two-chain as demonstrated by Page Blue 83 (BDH)-staining of SDS PAGE separated material.

### Example 4

### Plasminogen 1-544($asn_{289}$->gln;$thr_{345}$->ala)/t-PA 262-527(H41)

#### 4.1 Construction of plasmid pTRH41

Plasmid pTRH41 was constructed by ligating a single Ncol fragment of pTRH38 (Example 3) with an oligonucleotide linker (linker XVI).
Fragment XV : 8.0 kb Ncol fragment of pTRH38 which contains all the vector sequences
Linker XVI : was formed by annealing 4 oligonucleotides L,M,N and O of sequences :

L)    5' CATGGTGCCA TACAACCAAC AGCCAAGTGC GGTGGGAGTA
         CTGTAAGATA CCGTCCTGTG ACTCCTCCCC AGT 3'

M)    5' ATCCACGGAA CAATTGGCTC CCGCTGCACC ACCTGAGCTA
         ACCCCTGTGG TCCAGGACTG CTAC 3'

N)    5' CATGGTAGCA GTCCTGGACC ACAGGGGTTA GCTCAGGTGG
         TGCAGCGGGA GCCAATTGTT CCG 3'

O)    5' TGGATACTGG GGAGGAGTCA CAGGACGGTA TCTTACAGTA
         CTCCCACCGC ACTTGGCTGT TGGTTGTATG GCAC 3'

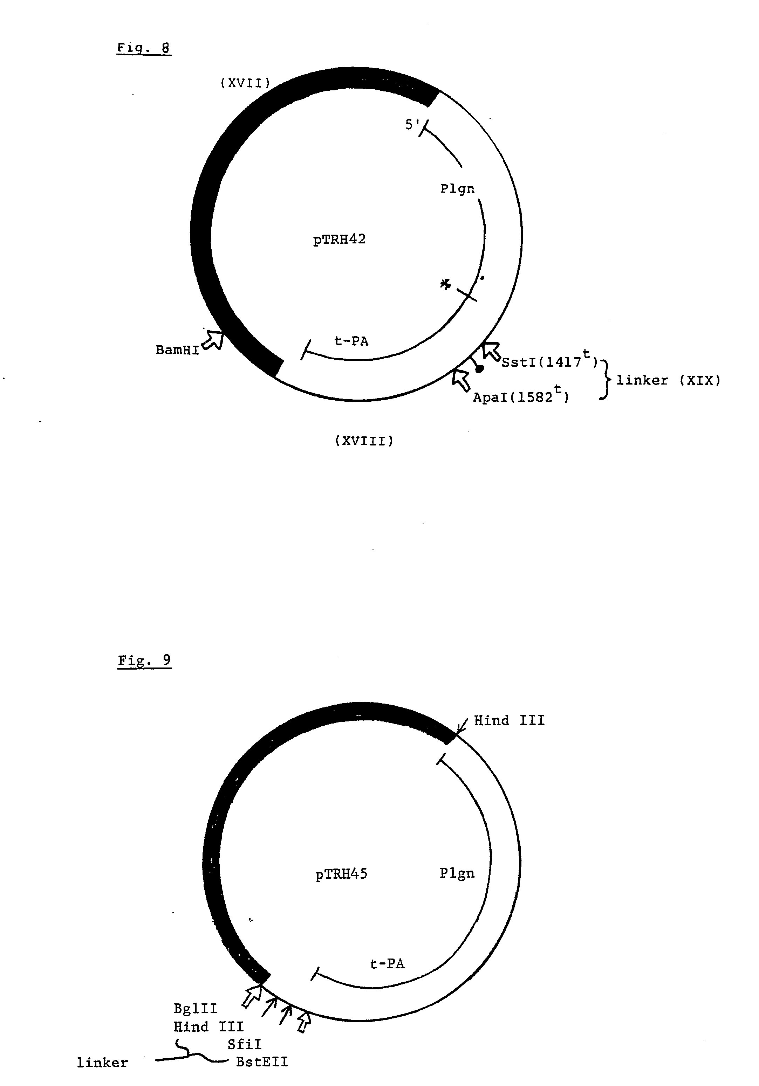

A plasmid (pTRH41) with the structure depicted in Fig. 7 was isolated. When human HeLa cells were transfected with plasmid pTRH41 a novel plasminogen activator (H41) was produced which had the same structure as H204 except that the N-linked glycosylation site at $asn_{289}$ and the O-linked glycosylation site at $thr_{346}$ in the plasminogen A-chain portion of the molecule are replaced by a glutamine and an alanine residue respectively. The protein is, therefore, not glycosylated at these sites.

### 4.2 Purification of protein H41

450ml conditioned medium from HeLa cells transfected with the plasmid pTRH41 was purified using Sephadex G25, zinc chelate Sepharose and lysine Sepharose and formulated essentially as described in Example 3.2. When assayed on fibrin plates the product contained 80,000 IU. Analysis by SDS-PAGE followed by fibrin zymography revealed a major fibrinolytic activity at $M_r$ of approximately 100,000. The protein content of the preparation was determined by Bradford assay (Anal. Biochem., 1976, 72 248-254) using bovine serum albumin as standard as 360ug.

### Example 5

### Plasminogen 1-544 ($asn_{289}$->gln;$thr_{346}$->ala)/t-PA 262-527 ($asn_{448}$->gln)(H42)

### 5.1 Construction of plasmid pTRH42

Plasmid pTRH42 was constructed by ligating 2 fragments of pTRH41 (Example 4) and pTRH204 (Reference Example 1) with an oligonucleotide linker (linker XIX).
Fragment XVII : 6.5 kb BamHI to SstI fragment of pTRH41 which contains most of the vector sequences. (SstI cuts in the $B^t$ domain at 1417).
Fragment XVIII : 1.5 kb ApaI to BamHI fragment of pTRH41 or equivalent plasmid, eg pTRH204 (ApaI cuts in the $B^t$ coding domain at 1582).
Linker XIX : was formed by annealing 4 oligonucleotides P,Q,R and S of sequences:

P)    5' CTCCGGCTAC GGCAAGCATG AGGCCTTGTC TCCTTTCTAT
         TCGGAGCGGC TGAAGGAGGC TCATGTCAGA CTGTACCCAT
         CCA 3'

Q) 5' GCCGCTGCAC ATCACAACAT TTACTTCAAC GCACAGTCAC
CGACAACATG CTGTGTGCTG GAGACACTCG GAGCGGCGGG
CC 3'

R) 5' CGCCGCTCCG AGTGTCTCCA GCACACAGCA TGTTGTCGGT
GACTGTGCGT TGAAGTAAAT GTTGTGATGT GCA 3'

S) 5' GCGGCTGGAT GGGTACAGTC TGACATGAGC CTCCTTCAGC
CGCTCCGAAT AGAAAGGAGA CAAGGCCTCA TGCTTGCCGT
AGCCGGAGAG CT 3'

A plasmid (pTRH42) with the structure depicted in Fig. 8 was isolated. Human HeLa cells, when transfected with plasmid pTRH42, produced a novel plasminogen activator (H42) which has the same structure as H204 except that the N-linked glycosylation site at $asn_{289}$ and the O-linked glycosylation site at $thr_{346}$ in the plasminogen A chain portion of the molecule are replaced by a glutamine and an alanine residue respectively and the N-linked glycosylation site at $asn_{448}$ in the B chain of the t-PA part of the molecule is, replaced by a glutamine residue. The protein is, therefore, believed to be completely non-glycosylated.

### 5.2 Purification of protein H42

510 ml conditioned medium from HeLa cells transfected with the plasmid pTRH42 was purified using Sephadex G25, zinc chelate Sepharose and lysine Sepharose and formulated essentially as described in Example 3.2. The product contained 17,000 IU (fibrin plate assay). Analysis by SDS-PAGE followed by fibrin zymography revealed a major doublet of fibrinolytic activity at apparent $M_r$ of approximately 85,000 and 95,000; the two species are believed to be the $glu_1$ and $lys_{78}$ N-terminal forms of the protein H42.

### Example 6

Construction of a pTRH37 derivative with a modified DNA sequence 3' to the coding region of the hybrid PA

This plasmid, pTRH45, is essentially the same as pTRH37 but with additional restriction sites introduced at the 3' end of the H37 coding region.

The large BglII to BstEII fragment of pTRH37 was ligated with oligonucleotides of sequence:-

T) 5' GTTACCAACTACCTAGACTGGATTCGTGACAACATGCGACCGTGAG
GCCTACTAGGCCAAGCTTA 3'

U) 5' GATCTAAGCTTGGCCTAGTAGGCCTCACGGTCGCATGTTGTCACG
AATCCAGTCTAGGTAGTTG 3'

These oligonucleotides introduce a unique SfiI site and a second Hind III site 3' to the H37 coding region. Plasmid pTRH45 is shown in Fig.9.

### Example 7

Plasminogen 1-541 (asn_{289}->gln; thr _{346}->ala)/ t-PA 262-527 (asn_{448}->gln) H42b)

The plasmid pTRH42b is designed to encode a non-glycosylated hybrid in which the amino acid sequence connecting the $K_5{}^P$ and $B^t$ domains is the same as encoded by plasmid pTRH37 (Reference Example 2) i.e.:

.....$K_5{}^P$]STCGLRQYSQPQFR[$B^t$]

Four fragments were ligated in the construction of pTRH42b (effectively replacing the 990bp Bst XI-SstI fragment of pTRH42 with the equivalent fragment from pTRH37):

Fragment XX : 5.5kb BamHI to BstXI fragment of pTRH41

Fragment XXI : 990bp BstXI to SstI fragment of pTRH37

Fragment XXII : 970bp BstEII to BamHI fragment of pTRH45

Fragment XXIII : 311bp SstI to BstEII fragment of pTRH42

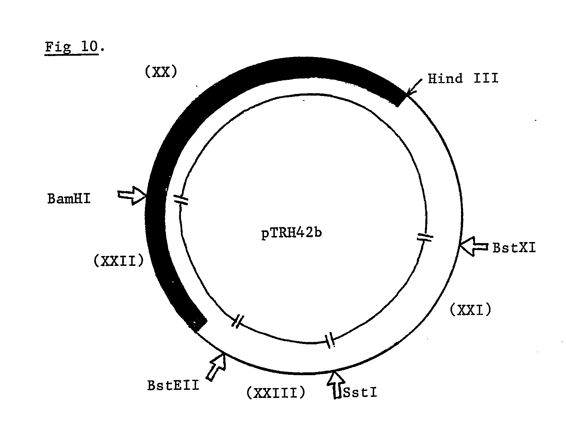

Fig. 10 shows the structure and derivation of plasmid pTRH42b.

The H42b protein was expressed as for H42 (Example 5). Medium from HeLa cells transfected with the plasmid pTRH42b was fibrinolytically active on human fibrin plates.

Example 8

Synthesis of deglyco plasminogen 1-541/t-PA 262-527 and deglyco plasminogen 78-541/t-PA 262-527

A purified preparation of tc H37 protein (37pmoles, 40μl), synthesised as described in Reference Example 2, was treated with Glycopeptidase F™ (1.5U, 7.5μl, Boehringer Mannheim) in a total incubation volume of 100μl in 25mM NaH2PO4/25mM EDTA/0.5% Triton X-100 pH 7.5 at 37°C for 24h. The product showed a similar dose response on fibrin plates to the native glycoprotein and was characterised as a deglycosylated tc H37 protein because

1) by SDS PAGE followed by fibrin zymography there was a small decrease in apparent $M_r$ of Glycopeptidase F-treated tc H37 protein compared to native material

2) by SDS PAGE under reducing conditions followed by Western blotting/Immunogold silver staining there was a small decrease in the $M_r$ of the t-PA B chain moiety of the Glycopeptidase F-treated tc H37 protein compared to the native material.

Example 9

Glu_{1} plasminogen (asn_{289}->gln; thr_{346}->ala)

9.1 Construction of intact (ile_{682}->val) plasminogen cDNA: plasmid pTRP1

A plasminogen cDNA clone (β) was isolated from the human liver cDNA library by screening with a [32P]-labelled overlapping BstXI restriction fragment (1209-1840) obtained from the partial plasminogen cDNA described in Reference Example 1. The new cDNA clone was sequenced and was found to encode the whole of the plasminogen B chain. The DNA sequence of the B chain is identical to Forsgren et al with two exceptions: the first is a G to A change at nucleotide 1821 which does not alter the encoded val_{563}, the second is an A to G change at nucleotide 2176 resulting in the encoded ile_{682} being changed to val. The 5' end of the clone is at nucleotide 1686 and the 3' end at nucleotide 2523 (Forsgren et al) i.e. the 3' end lies in the untranslated region.

A complete plasminogen cDNA clone was then assembled by ligating:

Fragment XXIV : 6.4 kb fragment of pTRH204; from the 3' BglII site to the BstXI site at 1209. (This fragment contains all the vector sequences).

Fragment XXV : 0.63kb BstXI fragment (1209-1840) from the partial plasminogen cDNA described in Reference Example 1.

Fragment XXVI : 0.7kb BstXI (1840) to EcoRI (site at 3' end of clone β at join with λ vector) fragment of isolated plasminogen B chain clone.

Linker (XXVII): was formed by heating together the 2 oligonucleotides (V and W) (26μl at 10μmolar

concentration) to 68°C for 5 minutes. They were allowed to anneal by cooling to room temperature for at least 15 minutes. The linker formed was kinased as methods section.

5′ AATTCACTCACCTAGAGA 3′　　(V)

5′ GATCTCTCTAGGTGAGTG 3′　　(W)

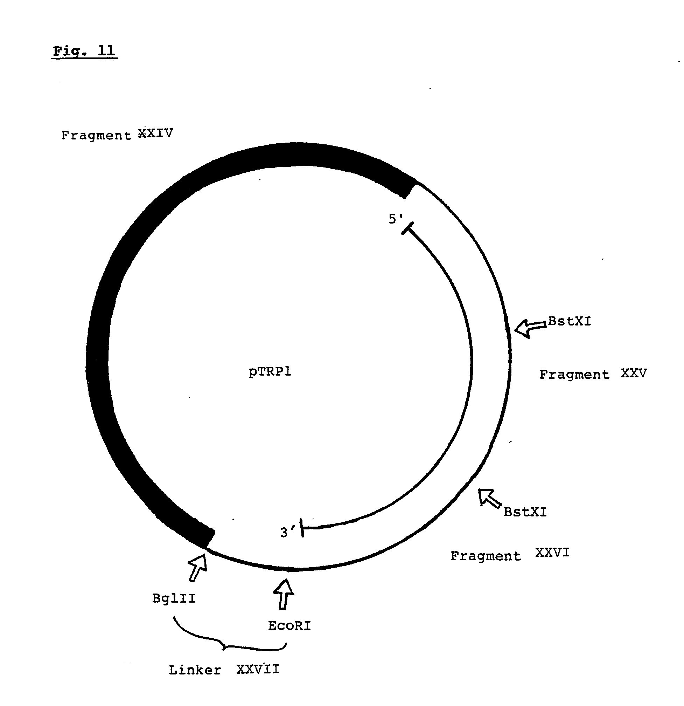

Plasmid pTRP1 was isolated (Fig. 11). It has a HindIII site in the 5′ untranslated region (as in pTRH204) and a BglII site in the 3′ untranslated region. pTRP1 was expressed in HeLa cells and encoded a protein of the expected size as determined by Western blotting using anti-plasminogen antibody.

9.2 Construction of 'consensus' plasminogen cDNA : plasmid pTRP2

The $val_{582}$ residue has not previously been reported. We, therefore, decided to convert the plasminogen cDNA clone to encode the plasminogen amino acid sequence as described by Sottrup-Jensen et al., Malinowski et al. and Forsgren et al. This was accomplished by in vitro mutagenesis and resulted in plasmid pTRP2.

pTRP2 was constructed by ligating together the following:

Fragment XXVIII: 7.4kb fragment of pTRP1 from the 3′ BglII site to the RsrII site at 2161. (This fragment contains all the vector sequences).

Fragment XXIX : 0.35kb fragment of pTRP1 from the 3′ BglII site to the StyI site at 2196.

Linker XXX : was formed by heating together the 2 oligonucleotides (X and Y) (10$\mu$molar) to 68°C for 5 minutes. They were allowed to anneal by cooling to room temperature for 15 minutes. The linker formed was kinased as methods section.

5′ GACCGAATGTTTCATCACTGGCTGGGGAGAAACC 3′　　(X)

5′ CTTGGGTTTCTCCCCAGCCAGTGATGAAACATTCG 3′　　(Y)

Plasmid pTRP2 was isolated. It has a HindIII site in the 5′ untranslated region (as in pTRH204) and a BglII site in the 3′ untranslated region. pTRP2 was expressed in HeLa cells and encoded a protein of the expected size as determined by Western blotting using anti-plasminogen antibody.

9.3 Construction of aglyco 'consensus' plasminogen cDNA: plasmid pTRP43

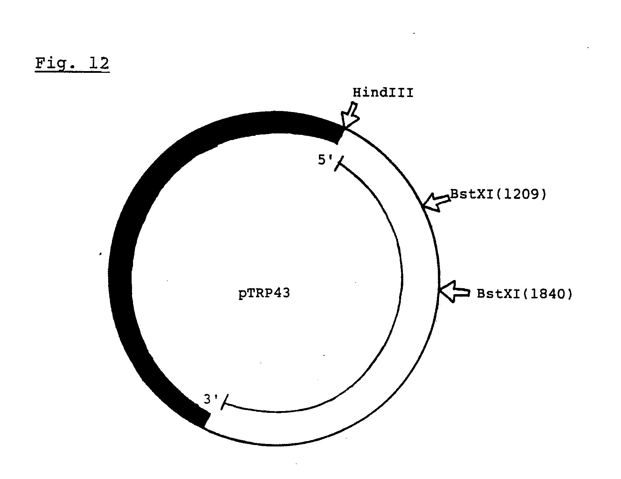

Plasmid pTRP43 was constructed by ligating the 6.6 kb BstXI-HindIII and 631 bp BstXI fragments of pTRP2 with the 1.16 kb Hind III->BstXI fragment of pTRH41 (Example 4). The 1.16kb HindIII-BstXI fragment from pTRH41 carries the $asn_{289}$->gln and $thr_{346}$->ala mutations in the plasminogen A-chain coding region. Plasmid pTRP43 (Fig. 12), when introduced into HeLa cells directed the synthesis of an aglyco 'consensus' plasminogen molecule (P43).

9.4 Purification of aglyco 'consensus' plasminogen (P43)

480 ml conditioned medium from HeLa cells transfected with the plasmid pTRP43 was purified using Sephadex G25, zinc chelate Sepharose and lysine Sepharose essentially as described in Example 3.2. Fractions from the lysine Separose column containing the P43 protein were identified using a streptokinase-based chromogenic substrate assay and were then formulated by ultra filtration followed by buffer-exchange into PST/10 mg ml$^{-1}$ mannitol/50$\mu$M $\epsilon$-ACA. When assayed against glu plasminogen in the streptokinase-based chromogenic substrate assay the product (2.5 ml) contained the equivalent of 64 nM plasminogen. Analysis by SDS-PAGE under non-reducing conditions followed by silver staining revealed three bands, the most intensely stained of which had an apparent $M_r$ of approximately 90,000.

Example 10

Synthesis of Streptokinase/aglyco 'consensus' plasminogen complex

25$\mu$l of conditioned medium from Example 9.3 were mixed with 25$\mu$l of streptokinase (Kabi) [20$\mu$M in 0.1M Triethanolamine pH 8.0/0.2mgml$^{-1}$ soybean trypsin inhibitor (Sigma) - TRIEN/SBT1] and incubated at 37°C for 15 min. 250$\mu$l of 1mM chromogenic substrate H-D-Valyl-Leucyl-Lysine. 2HCl (S-2251, Flow Laboratories) in TRIEN/SBT1 were then added and the solution incubated at 37°C for 24 h. Appropriate control conditioned media were assayed in parallel. $A_{405}$ values were determined using a Dynatech Minireader II microtitre plate reader. An increase in $A_{405}$ signifies the formation of a substance enzymatically active towards S-2251 and insensitive to SBT1. Since plasmin (a possible product of streptokinase action on plasminogen) is inhibited by SBT1, appearance of activity against S-2251 can be attributed to the SBT1-resistant streptokinase plasmin complex. By comparison of the control-corrected $A_{405}$ values obtained to a calibration curve of $A_{405}$ values vs. plasminogen concentration, (obtained by assaying preformed streptokinase/human lys-plasminogen (Immuno) complexes of known plasminogen concentration under the above conditions), the formation of a streptokinase/aglyco 'consensus' plasminogen complex was detected.

Example 11

Synthesis of 4-anisoyl aglyco consensus plasminogen 1-791(asn$_{289}$->gln; thr$_{346}$->ala)/streptokinase complex

Aglyco plasminogen (1.8 ml; 0.1 nmoles) purified as described in Example 9.4, in PST/10 mgm1$^{-1}$ mannitol/50 $\mu$M $\epsilon$-ACA pH7.4 containing 20% (w/v) glycerol, was treated with streptokinase (25 $\mu$l; 0.5 nmoles) and 4-amidinophenyl-4'-anisate. HCl (18 $\mu$l; 1.8 $\mu$moles) at 25°C for 60 min. The material was buffer-exchanged into PST/10 mgm1$^{-1}$ mannitol/50 $\mu$M $\epsilon$-ACA/20% (w/v) glycerol pH 7.4 (2.2 ml), aliquoted and stored at -40°C. On bovine fibrin plates the product gave 12.6 mU/ml using EMINASE (EMINASE is a trademark of Beecham Group plc) as a standard.

Example 12

Synthesis of 4-anisoyl aglyco plasminogen 78-791 asn$_{289}$->gln; thr$_{346}$->ala)/streptokinase complex

Aglyco plasminogen (1.5 ml; 40 pmoles), purified as described in Example 9.4, in PST/10 mgml$^{-1}$ mannitol/50 $\mu$M $\epsilon$-ACA pH 7.4, was treated with plasmin (30 $\mu$l; 0.4 pmoles) for 20 min at 37°C. This product was then treated with Streptokinase (10 $\mu$l; 200 pmoles), 4-amidinophenyl-4'-anisate. HCl (18 $\mu$l; 1.8 $\mu$moles) in 20% (w/v) glycerol for 60 min at 25°C. The material was buffer-exchanged into 0.05M NH$_4$HCO$_3$/10 $\mu$M $\epsilon$-ACA pH 7.6 and lyophilised. It was reconstituted in 100 $\mu$l ddH$_2$O and on bovine fibrin plates the product gave 0.26 MU/ml using EMINASE (EMINASE is a trademark of Beecham Group plc) as a standard.

Example 13

Deglyco plasminogen 78-546/u-PA 137-411

13.1 Construction of plasmid pTRH25

Plasmid pUC8 was digested with Hind III. The resulting approximately 2.7 kb vector fragment was ligated with an oligonucleotide linker XXXI composed of two oligonucleotides $\gamma$ and $\delta$ (see below) and transformed into E. coli HB101, and plasmid pTRHS was isolated. The DNA linker sequence shown in Table 4 encodes the polypeptide comprising amino acids 543-546 of plasminogen and 137-150 of urokinase.

## TABLE 4

### LINKER XXXI JOINING PLASMINOGEN AND UROKINASE SEQUENCES

```
                    plasminogen sequence | urokinase sequences
                    <─────────────────── | ───────────────────>

                              .... Phe | Pro Ser Ser Pro Pro ·
        γ: 5'  AGCTT GG GCG CCT TCA TTT | CCC TCC TCT CCT CCA
        δ:        3'A CC CGC GGA AGT AAA | GGG AGG AGA GGA GGT

                      ‿‿‿ ‿‿‿‿‿ ,.... 546 | 137 138 139 140 141


              Hind III   Nar I
```

```
        Glu Glu Leu ....
        GAA GAG CTC AAA TTT CAG TGT GGC CA GATCTA 3'
        CTT CTC GAG TTT AAA GTC ACA CCG GT CTAGATTCGA 5'
        142 143 144 ....        ‿‿‿‿   ‿‿‿‿‿‿  ‿.‿‿‿‿
               SstI             BalI    BglII    HindIII
```

Amino acids 143 and 144 of urokinase are coded by the nucleotide triplets GAG CTC rather than the native GAA TAA thus incorporating an SstI site. The linker also encodes a novel BglII site.

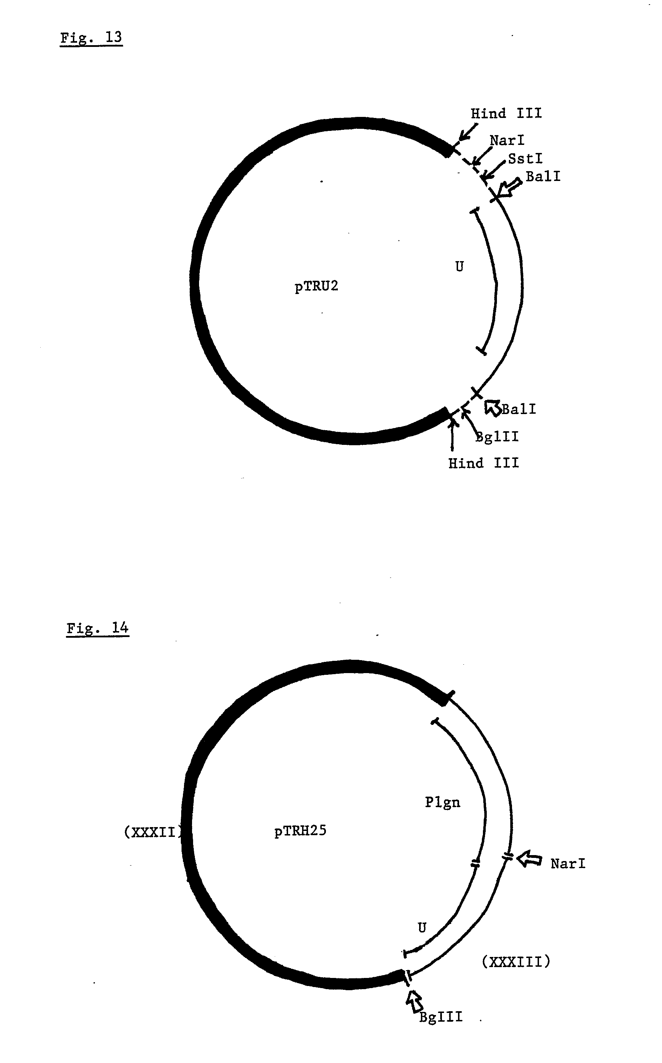

A human urokinase cDNA clone was identified by oligonucleotide probing and was isolated by conventional means (Holmes et al (1985) BioTechnology 3, 923-929). An approximately 1.5 kb BalI fragment was isolated from a partial digest of the cDNA clone; this fragment encodes most of the B-chain of urokinase and part of the 3′ untranslated region (corresponding to nucleotides 583 to 2154. It was ligated with BalI-digested pTRHS and transformed into E. coli HB101, and plasmid pTRU2 was isolated (Fig. 13).

To create the hybrid enzyme-coding sequence, plasmid pTRH9 (Reference Example 1) was digested with NarI and BglII and the approximately 7 kb fragment (XXXII) carrying vector functions and most of the plasmin A-chain coding region was isolated. Plasmid pTRU2 was digested with NarI and BglII and the approximately 1.5 kb (fragment XXXIII) (encoding part of the plasminogen A chain, all of the urokinase B-chain and part of the 3′ untranslated region) was isolated.

Fragments XXXII and XXXIII were ligated and transformed into E. coli. HB101 and plasmid pTRH25 isolated (Fig.14).

### 13.2 Purification of protein H25

510 ml conditioned medium, containing 500 KIU/ml aprotinin, from HeLa cells transfected with the plasmid pTRH25 was purified on Sephadex G25, zinc chelate Sepharose and lysine Sepharose and formulated essentially as described in Example 3.2. The major difference was that aprotinin (50 KIU/ml) was present in many of the chromatography buffers in an attempt to minimise nicking of the target protein.

When assayed on fibrin plates the dose response of the purified product was more closely parallel to that of t-PA rather than u-PA, and so results are expressed with reference to the International standard for t-PA. The product (2.5 ml) contained 110,000 IU. Analysis by SDS-PAGE under non-reducing conditions followed by staining for protein revealed a single major band at apparent $M_r$ of approximately 100,000. This band was coincident with the major fibrinolytic activity as determined by SDS-PAGE followed by fibrin zymography. When the product was analysed after storage at -40°C for approximately 2 months (with the occasional freeze/thaw cycle in the intervening period) it appeared to be mainly two-chain and in the $lys_{78}$ form.

### 13.3 Synthesis of deglyco plasminogen 78-546/u-PA 137-411

$lys_{78}$tc H25 protein from Example 13.2 (56 pmoles, 50 $\mu$l) was treated with Glycopeptidase FTM (0.6U, 3 $\mu$l, Boehringer Mannheim) in a total incubation volume of 100 $\mu$l in 25 mm $NaH_2PO_4$/25mM EDTA/0.5% Triton X-100 pH 7.5 at 37°C for 20h. The product showed a similar dose response on fibrin plates to the native glycoprotein and was characterised as a deglycosylated $lys_{78}$ tc H25 protein using techniques similar to those described in Example 1.

In the figures:

### Fig. 1 Structure of pTRH6

|  |  |
|---|---|
| ▬▬▬▬ | = pUC8 sequences |
| ———— | = plasminogen cDNA sequences |
| ⇨ | = restriction sites used in construction |
| ————> | = other restriction sites |

### Fig. 2 Structure of pTRH9

Fragments (I) to (III) and linker (IV) are indicated.

|  |  |
|---|---|
| ▬▬▬ | = pTRE12 D Nar Sequences |
| ———— | = insert Sequences |
| PlgnA | = plasminogen A chain coding region |
| ⇨ | = restriction sites used in construction |
| ————> | = other restriction sites |
| ⊢———⊣ | = protein coding regions |

### Fig. 3 Structure of pTRH20

= vector sequences

= insert DNA

= restriction sites used in construction

——> = other restriction sites

* = linking amino acid sequence

|——⌐ = protein coding regions

PlgnA = plasminogen A chain-coding region

$A^t$ = t-PA A chain coding region

$B^t$ = t-PA B chain coding region

Fig. 4 Structure of pTRH204

Fragments (V) (SstI-NarI), (VI)(BanII-SstI) and linker (VII)(NarI-BanII) are indicated.

= vector sequences

= insert DNA

= restriction sites used in construction

——> = other restriction sites

* = serine equivalent to plasminogen serine
545/t-PA serine 262 at junction

|——⌐ = protein coding regions

PlgnA = plasminogen A chain coding region ($glu_1$ to
$pro_{544}$)

t-PA = t-PA coding region ($thr_{263}$ to $pro_{527}$)

Fig 5 Structure of pTRH37

Fragments (VI) (BanII-SstI), (VIII) (SstI-BstXI), (IX) (BstXI-AvaII) and linker (X) (AvaII-BanII) are indicated.

= vector sequences

= insert DNA

= restriction sites used in construction

——> = other restriction sites

|——⌐ = protein coding regions

Plgn = plasminogen sequences ($glu_1$-$cys_{541}$)

t-PA = t-PA sequences ($ser_{262}$ to $pro_{527}$)

Fig. 6 Structure of pTRH38

Fragments (XI)(NcoI-AvaIII),(XII)(AvaIII-SnoI),(XIII) (Nco1 fragment) and linker (XIV)(SnoI-NcoI) are indicated.

     ▬▬▬     = vector sequences

    ─────────    = insert DNA

    ⟹    = restriction sites used in construction

   ⊢────⊣   = protein coding regions

   Plgn = plasminogen coding region ($glu_1$-$pro_{544}$, with $asn_{289}$->$gln$ change).

   t-PA = t-PA coding region ($thr_{263}$-$pro_{527}$)

      * = serine equivalent to plasminogen serine545 or t-PA serine262 at junction

   ────────● = position of altered amino acid ($asn_{289}$->$gln$)

## Fig. 7 Structure of pTRH41

Fragments (XV) (NcoI fragment) and linker (XVI) are indicated.

     ▬▬▬     = vector sequences

    ─────────    = insert DNA

    ⟹    = restriction sites used in construction

      * = serine equivalent to plasminogen serine 545/t-PA serine 262 at junction

   ⊢────⊣   = protein coding regions

   Plgn = plasminogen sequences ($glu_1$-$pro_{544}$ with $asn_{289}$->$gln$ change and $thr_{346}$->$ala$ change)

   t-PA = t-PA coding sequences ($thr_{263}$-$pro_{527}$)

   ────────● = position of altered amino acid ($thr_{346}$->$ala$)

## Fig. 8 Structure of pTRH42

Fragments (XVII) (BamHI-SstI), (XVIII) (ApaI-BamHI) and linker (XIX)(SstI-ApaI) are indicated.

28

███ = vector sequences

─────── = insert DNA

⇒ = restriction sites used in construction

\* = serine equivalent to plasminogen serine 545/t-PA serine 262 at junction

├───┤ = protein coding regions

Plgn = plasminogen sequences ($glu_1$-$pro_{544}$ with $asn_{289}$->$gln$ and $thr_{346}$->$ala$ changes)

t-PA = t-PA sequences ($thr_{263}$-$pro_{527}$ with $asn_{448}$->$gln$ change)

───────● = position of altered amino acid ($asn_{448}$->$gln$)

Fig. 9 Structure of pTRH45

███ = vector sequences

─────── = insert DNA

⇒ = restriction sites used in construction

───────> = other restriction sites

├───┤ = protein coding region

Plgn = plasminogen sequences ($glu_1$-$cys_{541}$)

t-PA = t-PA sequences ($thr_{263}$ to $pro_{527}$)

Fig.10 Structure of pTRH42b

Fragments (XX), (XXI), (XXII) and (XXIII) are indicated

███ = vector sequence

─────── = insert DNA

⇒ = restriction sites used in construction

───────> = other restriction sites

├───┤ = origin of fragments used in construction

Fig. 11 Structure of pTRP1

Fragments (XXIV), (XXV), (XXVI) and linker (XXVII) are indicated.

▬▬▬▬ = vector sequences

――――――― = insert DNA

⇒ = restriction sites used in construction

├――――┤ = plasminogen coding region

Fig. 12 Structure of pTRP43

▬▬▬▬ = vector sequence

――――――― = insert DNA

⇒ = restriction sites used in construction

├――――┤ = plasminogen coding region
($glu_1$-$asn_{791}$ with $asn_{289}$->gln and
$thr_{346}$->ala changes)

Fig. 13 Structure of pTRU2

▬▬▬▬ = vector sequences

――――――― = insert DNA

------- = introduced linker sequence

⇒ = restriction sites used in construction

―――――> = other restriction sites

├――――┤ = protein coding sequences

U = Urokinase sequences ($pro_{137}$ - $leu_{411}$)

Fig. 14 Structure of pTRH25

Fragments (XXXII) and (XXXIII) are indicated.

▬▬▬▬ = vector sequences

―――――> = insert DNA

⇒ = restriction sites used in construction

├――――┤ = protein coding regions

Plgn = plasminogen sequences ($glu_1$ - $phe_{546}$)

U = urokinase sequences ($pro_{137}$ - $leu_{411}$)

**Claims**

1. Plasminogen or a plasminogen activator containing the five kringle domains of plasminogen in which a carbohydrate structure cannot be attached to amino acid 289.

2. Plasminogen or a plasminogen activator according to claim 1 which comprises an amino acid residue other than asparagine at position 289.

3. Plasminogen or a plasminogen activator according to claim 2 wherein the amino acid residue at position 289 is glutamine or aspartic acid.

4. Plasminogen or a plasminogen activator according to any preceding claim, which comprises an amino acid residue other than threonine at position 346.

5. A plasminogen activator according to claim 1 selected from:

p-anisoyl streptokinase-lys plasminogen (asn$_{289}$->gln; thr$_{346}$->ala) activator complex with substantially no internal peptide bond cleavage;

p-anisoyl streptokinase-glu plasminogen (asn$_{289}$->gln; thr$_{346}$->ala) activator complex with substantially no internal peptide bond cleavage; or

a hybrid plasminogen activator selected from

Plg 1-541(asn$_{289}$->gln)[AAPSTCGLRQYSQPQFR]t-PA 276-527,

Plg 1-541(asn$_{289}$->gln); thr$_{346}$->ala) [AAPSTCGLRQYSQPQFR]t-PA 276-527,

Plg 1-541(asn$_{289}$->gln); thr$_{346}$->ala) [AAPSTCGLRQYSQPQFR]t-PA 276-527(asn$_{448}$->gln),

Plg 1-541(asn$_{289}$->gln); thr$_{346}$->ala) [STCGLRQYSQPQFR]t-PA 276-527(asn$_{448}$->gln),

Deglyco plg 1-541[AAPSTCGLRQYSQPQFR]t-PA 276-527 (product of the treatment of the glycosylated compound with peptide:N-glycosidase F),

Deglyco plg 1-541[STCGLRQYSQPQFR]t-PA 276-527 (product of the treatment of the glycosylated compound with peptide:N-glycosidase F) and

Deglyco plg 1-541[AAPSFPSSPPEELKFQCGQKTLRPRFK] u-PA 159-411 (product of the treatment of the glycosylated compound with peptide:N-glycosidase F),

where plg 1-541 represents residues 1-541 of plasminogen (that is, including kringles 1 to 5) and the symbols in brackets represent amino acid residues according to the single letter amino acid notation, including one and two chain variants, lys$_{78}$ and glu$_1$ variants, and mixtures thereof.

6. A plasminogen activator according to any preceding claim wherein any catalytic site essential for fibrinolytic activity is optionally blocked by a removable blocking group.

7. A plasminogen activator according to claim 6 wherein the removable blocking group is 4-chloro-2-aminobenzoyl.

8. A process for preparing plasminogen or a plasminogen activator according to any one of claims 1 to 7, which process comprises :

(a) treating the corresponding protein having a carbohydrate structure at position asn$_{289}$ with peptide; N-glycosidase F, or

(b) expressing DNA encoding said protein in a recombinant host cell and recovering the protein product; and thereafter optionally forming a complex of modified plasminogen with streptokinase and/or optionally blocking any catalytic site essential for fibrinolytic acitivity with a removable blocking group.

9. A pharmaceutical composition which comprises plasminogen or a plasminogen activator according to any of claims 1 to 7 in combination with a pharmaceutically acceptable carrier.

10. The use of a plasminogen or a plasminogen activator according to any of claims 1 to 7 for the manufacture of a medicament for the treatment of thrombotic diseases.

Fig. 1

Fig. 2

Fig. 3

Fig. 4

Fig. 5

Fig. 6

AvaIII(631)
SnoI(974)    linker (XIV)
NcoI(1072)
NcoI(1209)

(XI)    pTRH38    (XII)    (XIII)

5'    Plgn

3'    t-PA

Fig. 7

NcoI(1072)    linker (XVI)
NcoI(1209)

(XV)    pTRH41

5'    Plgn

3'    t-PA

Fig. 8

(XVII)

5'

Plgn

pTRH42

t-PA

BamHI

SstI(1417$^t$)
ApaI(1582$^t$)
} linker (XIX)

(XVIII)

Fig. 9

Hind III

pTRH45

Plgn

t-PA

BglII
Hind III
SfiI
BstEII

linker

Fig 10.

(XX)

Hind III

BamHI

pTRH42b

BstXI

(XXII)

(XXI)

BstEII

(XXIII) SstI

**Fig. 11**

Fragment XXIV

pTRP1

5'

BstXI

Fragment XXV

BstXI

Fragment XXVI

3'

BglII

EcoRI

Linker XXVII

Fig. 12

Fig. 13

Fig. 14

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | EP-A-0 290 118 (BEECHAM GROUP) <br> * Claims * <br> --- | 1-10 | C 12 N 9/64 <br> C 12 N 9/68 <br> C 12 N 15/58 <br> A 61 K 37/547 <br> C 12 N 15/57 <br> C 12 N 15/62 |
| A | EP-A-0 213 794 (AMERICAN HOME PRODUCTS) <br> * Claims * <br> --- | 1-10 | |
| D,P <br> A | EP-A-0 297 882 (BEECHAM GROUP) <br> * Claims * <br> --- | 1-10 | |
| A | EP-A-0 277 313 (CIBA-GEIGY) <br> * Claims * <br> --- | 1-10 | |
| A | BIOCHEMISTRY, vol. 24, 1985, pages 4665-4671; A.L. TARENTINO et al.: "Deglycosylation of asparagine-linked glycans by peptide:N-Glycosidase F" <br> * Whole article * <br> ----- | 1,5,8 | |

TECHNICAL FIELDS
SEARCHED (Int. Cl.5)

C 12 N

The present search report has been drawn up for all claims

| Place of search | Date of completion of the search | Examiner |
|---|---|---|
| THE HAGUE | 05-03-1990 | HUBER-MACK A. |

EPO FORM 1503 03.82 (P0401)